# EUROPEAN PATENT SPECIFICATION

(11) **EP 3 074 440 B1**
(45) Date of publication and mention of the grant of the patent: **27.02.2019**
(21) Application number: 14865291.0
(22) Date of filing: 04.07.2014
(51) Int. Cl.: C08F 293/00, C08F 120/00, A61K 47/30

(54) **MIKTO-ARM BRANCHED POLYMERS**
VERZWEIGTE MIKRO-ARM-POLYMERE
POLYMÈRES RAMIFIÉS À BRANCHES MIKTO

(30) Priority: 28.11.2013 AU 2013904605
(43) Date of publication of application: 05.10.2016
(73) Proprietor: Commonwealth Scientific and Industrial Research Organisation, Acton, ACT 2601 (AU)
(72) Inventor: GUNATILLAKE, Pathiraja Arachchillage, Mulgrave, Victoria 3170 (AU); HINTON, Tracey Michelle, Belmont, Victoria 3216 (AU); THANG, San Hoa, Camberwell, Victoria 3124 (AU)
(74) Representative: Engelhard, Markus
(86) International application number: PCT/AU2014/050113
(87) International publication number: WO 2015/077831

(56) References cited:
- WO-A1-2010/021770
- WO-A1-2013/003887
- WO-A1-2013/113071
- WO-A1-2013/113071
- CN-B- 101 143 916
- ZHANG, Y: "One pot synthesis of ABC miktoarm star terpolymers by coupling ATRP, ROP, and click chemistry techniquesOne-pot synthesis of", JOURNAL OF POLYMER SCIENCE PART A: POLYMER CHEMISTRY, vol. 42, no. 12, 17 January 2009 (2009-01-17), pages 3066-3077, XP002770279, wiley
- BLENCOWE, A. ET AL.: 'Core cross-linked star polymers via controlled radical polymerisation' POLYMER vol. 50, 2009, pages 5 - 32, XP025866370
- WEI, X. ET AL.: 'Synthesis of cleavable multi-functional mikto-arm star polymer by RAFT polymerization: example of an anti-cancer drug 7-ethyl-10-hydroxycamptothecin (SN-38) as functional moiety' SCIENCE CHINA , CHEMISTRY vol. 57, no. 7, July 2014, pages 995 - 1001, XP055345910

## Description

### Field of the Invention

The present invention relates in general to mikto-arm branched polymers. In other words, the invention relates in general to branched polymers having at least three different polymer arms. The mikto-arm branched polymers are particularly suited for use in forming complexes with nucleic acid molecules, and it will therefore be convenient to describe the invention with an emphasis toward this application. However, it is to be understood that the mikto-arm branched polymers may be used in various other applications. The invention therefore also relates to a complex of a nucleic acid molecule and the mikto-arm branched polymer, to the use of such complexes in a method of delivering a nucleic acid molecule to cells, and to a method of silencing gene expression. The invention further relates to the use of the mikto-arm branched polymer in a method of protecting a nucleic acid molecule from enzymatic degradation.

### Background of the Invention

Branched polymers are a type of polymer known in the art to comprise a support moiety such as an atom or molecule to which is attached at least three polymer chains. The at least three polymer chains may be referred to as the "arms" of the branched polymer. Specific types of branched polymer include star polymers, comb polymers, brush polymers and dendrimers.

Branched polymers have been found to exhibit a variety of unique properties and have been employed in a diverse array of applications functioning, for example, as elastomers, surfactants, lubricants and delivery agents for bioactives.

ZHANG, Y: "One pot synthesis of ABC miktoarm star terpolymers by coupling ATRP-ROP, and click chemistry techniques", Journal of Polymer Science Part A: Polymer Chemistry, vol. 42, no. 12, 1 January 2009, pages 3066-3077, discloses one-pot synthesis of ABC mikto-arm star terpolymers by coupling ATRP, ROP, and click chemistry techniques.

WO 2013/113071 A1 relates to branched polymers comprising a support moiety and at least three block co-polymer chains.

WO 2010/021770 A1 relates to compositions comprising a heterogeneous polymeric micelle and an agent as well as methods for intracellular delivery.

WO 2013/003887 A1 discloses nucleic acid complexes comprising a cationic block copolymer and a nucleic acid.

The delivery of bioactives remains a challenge as there are numerous variables that effect the outcome such as the bioactive stability, release rates, release triggers and biocompatability.

Accordingly, there remains an opportunity for developing new branched polymer structures that exhibit properties suitable for further extending the utility of this class of polymer.

### Summary of the Invention

The present invention provides branched polymer comprising a support moiety and at least three homopolymer chains each covalently coupled to and extending from the support moiety, wherein the at least three homopolymer chains include a cationic homopolymer chain, a hydrophilic homopolymer chain, and a hydrophobic homopolymer chain.

It has now been found that branched polymers according to the present invention provide a unique combination of at least cationic, hydrophilic and hydrophobic homopolymer features that surprisingly enable them to not only efficiently form a complex with a nucleic acid molecule but also enhance serum stability and cell uptake of the resulting complex.

Each of the at least three homopolymer arms attached to the support moiety are different and the branched polymers may therefore also be referred to as mikto-arm branched polymers.

WO 2013/113071 discloses branched polymer comprising a support moiety and at least three block co-polymer chains covalently coupled to and extending from the moiety. The block co-polymer chains each comprise a hydrophilic polymer block and a cationic polymer block, and optionally a hydrophobic polymer block. Surprisingly, despite having similar polymeric components to the branched polymer disclosed in WO 2013/113071, the mikto-arm homopolymer structure of branched polymers according to the present invention demonstrate improved properties over the branched block co-polymer structures disclosed in WO 2013/113071.

For example, the mikto-arm homopolymer structure of branched polymers according to the present invention demonstrate improved gene silencing properties. Such improved properties include gene silencing at low dosages. For example, effective gene silience has been obtained at siRNA concentrations as low as 50 nM compared with siRNA concentrations of at least 250 nM using branched block co-polymer structures disclosed in WO 2013/113071. Use in multiple cell lines has also been demonstrated indicating versatility. The mikto-arm homopolymer structure of branched polymers according to the present invention can also be more readily functionalised at the end of an arm compared with a multi-step approach required to achieve the same goal with the branched block co-polymer structures disclosed in WO 2013/113071.

In one embodiment, the branched polymer is a mikto-arm star polymer.

In another embodiment, one or more of the at least three homopolymer chains is covalently coupled to the support moiety through a degradable functional group.

Degradation of the degradable functional group results in the covalent coupling being cleaved and the homopolymer chain being severed from the support moiety.

Each of the at least three homopolymer chains may be covalently coupled to the support moiety through a degradable functional group.

In another embodiment, the support moiety has a molecular structure comprising one or more degradable functional groups which upon undergoing degradation result in cleavage of one or more covalent bonds in that molecular structure and release of at least one of the at least three the homopolymer chains from the support moiety.

Loss from the branched polymer of one or more of homopolymer chains can advantageously promote a change in the polymer's properties such as its hydrophilic, hydrophobic, and/or cationic character. The molecular weight of the branched polymer will of course also be reduced.

Through appropriate selection and location of degradable functional groups, the branched polymer can be designed to undergo specific structural transformations in a particular degrading environment.

In one embodiment, the degradable functional groups are biodegradable functional groups.

For example, the branched polymer may be designed to form a complex with a nucleic acid molecule, where upon administration of the complex and subsequent transfection the suitably located biodegradable functional groups undergo biodegradation resulting in separation of one or more of the homopolymer chains from the branched polymer structure. This structural transformation of the branched polymer within the cell may provide for enhanced availability of the nucleic acid molecule and also facilitate metabolism and clearance of the branched polymer (and/or its residues).

The present invention therefore also provides a complex comprising a branched polymer and a nucleic acid molecule, the branched polymer comprising a support moiety and at least three homopolymer chains each covalently coupled to and extending from the moiety, wherein the at least three homopolymer chains include a cationic homopolymer chain, a hydrophilic homopolymer chain, and a hydrophobic homopolymer chain.

In this context, it will be appreciated that the complex *per se* is formed between the branched polymer and the nucleic acid molecule.

The branched polymer can form stable complexes with a variety of nucleic acid molecules, with the resulting complex affording improved transfection for a nucleic acid molecule to a variety of cell types. The branched polymers, when in the form of the complex, have also been found to afford good protection to nucleic acid molecules from enzymatic degradation.

Each of the at least three homopolymer arms of the branched polymer can advantageously be tailor-designed to provide for efficient complexation with a given nucleic acid molecule and/or for efficient transfection of the nucleic acid molecule with a given cell type. The branched polymer and/or nucleic acid molecule can also advantageously be tailor-designed to incorporate a targeting ligand that directs the complex to a chosen targeted cell type.

In one embodiment, the branched polymer and/or nucleic acid molecule is conjugated with a targeting ligand.

In another embodiment, the branched polymer has more than one cationic homopolymer chain, hydrophilic homopolymer chain, or hydrophobic homopolymer chain covalently coupled to the support moiety.

The present invention also provides a method of delivering a nucleic acid molecule to a cell, the method comprising:
(a) providing a complex comprising a branched polymer and a nucleic acid molecule, the branched polymer comprising a support moiety and at least three homopolymer chains each covalently coupled to and extending from the moiety, wherein the at least three homopolymer chains include a cationic homopolymer chain, a hydrophilic homopolymer chain, and a hydrophobic homopolymer chain; and
(b) delivering the complex to the cell.

In one embodiment, the nucleic acid molecule is delivered to a cell for the purpose of silencing gene expression.

The present invention therefore also provides a method of silencing gene expression, the method comprising transfecting a cell with a complex comprising a branched polymer and a nucleic acid molecule, the branched polymer comprising a support moiety and at least three homopolymer chains each covalently coupled to and extending from the moiety, wherein the at least three homopolymer chains include a cationic homopolymer chain. a hydrophilic homopolymer chain, and a hydrophobic homopolymer chain.

In one embodiment of this and other aspects of the invention, the nucleic acid molecule is selected from DNA and RNA.

In a further embodiment, the DNA and RNA are selected from gDNA, cDNA, double or single stranded DNA oligonucleotides, sense RNAs, antisense RNAs, mRNAs, tRNAs, rRNAs, small/short interfering RNAs (siRNAs), double-stranded RNAs (dsRNA), short hairpin RNAs (shRNAs), piwi-interacting RNAs (PiRNA), micro RNA/small temporal RNA (miRNA/stRNA), small nucleolar RNAs (SnoRNAs), small nuclear (SnRNAs) ribozymes, aptamers. DNAzymes, ribonuclease-type complexes, hairpin double stranded RNA (hairpin dsRNA), miRNAs which mediate spatial development (sdRNAs), stress response RNA (srRNAs), cell cycle RNA (ceRNA-s) and double or single stranded RNA oligonucleotides.

Branched polymers in accordance with the invention have also been found to protect nucleic acid molecules against enzymatic degradation.

The present invention therefore also provides a method of protecting a nucleic acid molecule form enzymatic degradation, the method comprising complexing the nucleic acid molecule with a branched polymer comprising a support moiety and at least three homopolymer chains each covalently coupled to and extending from the moiety, wherein the at least three homopolymer chains include a cationic homopolymer chain, a hydrophilic homopolymer chain, and a hydrophobic homopolymer chain.

There is also provided use of a complex for delivering a nucleic acid molecule to a cell, the complex comprising a branched polymer and the nucleic acid molecule, the branched polymer comprising a support moiety and at least three homopolymer chains each covalently coupled to and extending from the moiety, wherein the at least three homopolymer chains include a cationic homopolymer chain, a hydrophilic homopolymer chain, and a hydrophobic homopolymer chain.

The present invention further provides use of a complex for silencing gene expression, the complex comprising a branched polymer and a nucleic acid molecule, the branched polymer comprising a support moiety and at least three homopolymer chains each covalently coupled to and extending from the moiety, wherein the at least three homopolymer chains include a cationic homopolymer chain, a hydrophilic homopolymer chain, and a hydrophobic homopolymer chain.

The present invention still further provides use of a branched polymer in protecting a nucleic acid molecule from enzymatic degradation, the branched polymer comprising a support moiety and at least three homopolymer chains each covalently coupled to and extending from the moiety, wherein the at least three homopolymer chains include a cationic homopolymer chain, a hydrophilic homopolymer chain, and a hydrophobic homopolymer chain.

Further aspects and embodiments of the invention appear below in the detailed description of the invention.

### Brief Description of the Drawings

The invention will herein be described with reference to the following non-limiting drawings in which:
Figure 1 illustrates a schematic representation of a branched polymer according to the invention;
Figure 2 illustrates a schematic representation of a method for preparing a branched polymer according to the invention;
Figure 3 illustrates (A) semi-logarithmic plot and (B) Evolution of number-average molecular weight and dispersity with monomer conversion for the linear polymerization of poly[(oligo(ethyleneglycol methacrylate)], poly[2-(dimethylamino)ethyl methacrylates]. poly(*n*-bulyl methacrylate) via RAFT using 4-cyano-4-((dodecylsulfanylthiocarbonyl)sulfanyl]pentanoic acid as chain transfer agent at a relative molar ratio of [RAFT]:[ACCN] 1.0:0.3 in DMF at 90°C;

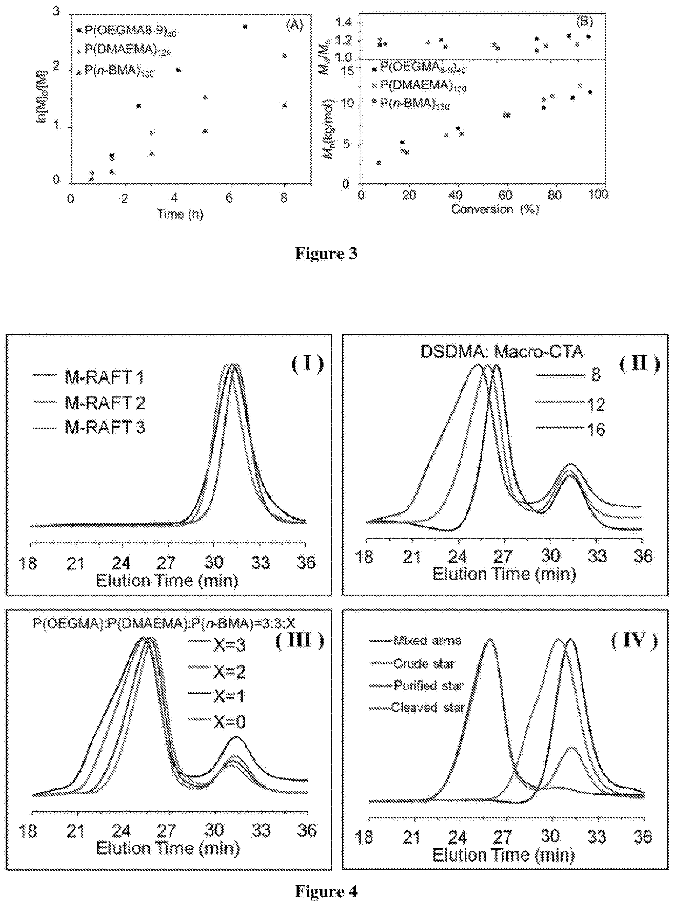

Figure 4 (I) illustrates the GPC traces obtained for the three macro-RAFT agents with P(OEGMA₈₋₉), P(DMAEMA) and P(*n*-BMA), respectively (denoted as M-RAFT 1, 2, 3 Figure 4 I); Figure 4 (II) illustrates the GPC traces of the mikto-arm star polymers synthesized with different [cross linker/macro-RAFT] ratio = 8, 12, 16; Figure 4 (III) the GPC traces of the mikto-arm star polymers synthesized with different P(*n*-BMA) molar ratios: P(OEGMA₈₋₉) / P(DMAEMA) / P(*n*-BMA) = 3/3/x, with x = 3, 2, 1, 0; and Figure 4 (IV) illustrates the GPC traces of the crude star polymers, purified star polymer, mixed arm polymers, and cleaved star polymers;
Figure 5 shows the ¹H NMR spectrum of purified mikto-arm polymerS3-6 (see Table 2 for details);
Figure 6 shows the ¹H NMR spectrum of mikto-arm star polymer and quanternized mikto-arm star polymer S3-6 (see Table 2 for details);
Figure 7 illustrates the association of mikto-arm polymers with siRNA as a function of polymer:siRNA ratio (w/w) for series of polymers prepared in Example 1 experimental results: (I) polymer alone, (II) N:P ratio 2, (III) 5, and (IV) 10;
Figure 8 illustrates the release of siRNA under reductive conditions (TCEP);
Figure 9 illustrates the cell viability of mikto-arm star polymers prepared in Example lin Huh7, A549 and CHO-GFP cell lines at different polymer concentrations;
Figure 10 illustrates the cell viability of mikto-arm polymers at different concentrations used for silencing experiments in cell lines CHO-GFP, A549 and Huh7;
Figure 11 illustrates gene silencing in CHO-GFP cells as measured by % GFP mean fluorescence of mikto-arm polymers prepared as described in Example 1 at N:P ratios of 2 and 1;
Figure 12 illustrates COPA silencing in A549 and Huh7 cells by mikto-arm polymers S4-1 and S4-5 at concentrations of 125 and 50 nM; and
Figure 13 illustrates the delivery of the cancer drug SN38 to Huh7 cells using the mikto-arm polymer S4-4.
Figure 14 illustrates the internalization of siRNA (green) and labelled (red) particles in the cytoplasm of CHO cells using confocal micrography demonstrating the internalisation; and
Figure 15 illustrates the gene silencing in CHO-GFP cells with 4-arm star block copolymers prepared according to methods described in WO 2013/113071: TL38-50 (50% quaternized), TL38-100 (100% quaternized), TL-84 (100% quaternized without PolyFluor), and TL-85 (100% quaternized, with Polyfluore).
Figure 16 illustrates fold change in ssB mRNA expression by qRT_PCR analysis A) Spleen B) Kidney C) Lung D) Liver. Data was analysed by repeated measures ANOVA with Tukey post analysis compared to PBS controls. ** p<0.05, *** p<0.05, NS Not significant.

Some Figures contain colour representations or entities. Coloured versions of the Figures are available upon request.

### Detailed Description of the Invention

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

As used herein, the singular forms "a", "and" and "the" are intended to include plural aspects unless the context clearly dictates otherwise. Thus, for example, reference to "a cell" includes a single cell as well as two or more cells; reference to "an agent" includes one agent, as well as two or more agents; and so forth.

As used in the context of the present invention the expression "branched polymer" is intended to mean polymer that comprises a support moiety to which is attached at least three homopolymer chains. The branched polymer may comprise more than one of such support moieties.

Each of the at least three homopolymer arms attached to the support moiety are different and the branched polymers may therefore also be referred to as mikto-arm branched polymers.

Specific types of branched polymer include, but are not limited to, star polymers, comb polymers, brush polymers and dendrimers.

The branched polymers according to the present invention have at least one cationic homopolymer chain, at least one hydrophilic homopolymer chain and at least one hydrophobic homopolymer chain each covalently coupled to a support moiety. For convenience, the at least three homopolymer chains covalently coupled to the support moiety may be referred to as "arms" of the branched polymer.

In one embodiment, the branched polymer is a mikto-arm star polymer.

The branched polymer may have more than three arms. For example, the branched polymer may have 4, 5, 6, 7, 8, 9, 10 or more homopolymer chains attached to the support moiety.

In one embodiment, the branched polymer has more than one (i) cationic homopolymer chain, (ii) hydrophilic homopolymer chain, and/or (ii) hydrophobic homopolymer chain covalently coupled to the support moiety.

In another embodiment, the ratio of cationic homopolymer chain(s), hydrophilic homopolymer chain(s), and hydrophobic homopolymer chain(s) is about 1:1:1.

Provided the at least three homopolymer chains are each covalently coupled to the support moiety, the branched polymer may also have one or more copolymer chains covalently coupled to the support moiety.

As used herein a "homopolymer" chain is intended to mean a polymer chain having a molecular structure that is derived from the polymerised residues of the same monomer.

As used herein a "copolymer" chain is intended to mean a polymer chain having a molecular structure that is derived from the polymerised residues of at least two different monomers.

The at least three homopolymer chains attached to the support moiety may each independently be branched or linear homopolymer chains.

The branched polymer according to the invention may have a combination of branched and linear homopolymer chains.

By homopolymer chains attached to the support moiety being "branched" is meant the homopolymer chain presents pendant groups from the main polymer backbone which have a branched structure. For example, a branched homopolymer chain may be derived from monomer that comprises a branched oligo ethylene oxide moiety (e.g. an oligio (ethylene glycol) methacylate) and thereby presents branched oligo ethylene oxide groups pendant from the main polymer backbone.

In one embodiment, the at least three homopolymer chains attached to the support moiety are linear homopolymer chains.

In a further embodiment, the branched polymer in accordance with the invention is a star polymer.

By "star polymer" is meant a macromolecule comprising a single branch moiety from which emanate at least three covalently coupled polymer chains or arms. According to the present invention (i) that branch moiety represents the support moiety, and the support moiety may be in the form of a suitable atom, molecule or molecular structure as herein described, and (ii) the at least three covalently coupled polymer chains or arms are the at least three homopolymer chains.

Where the branched polymer according to the invention is a star polymer, the at least three homopolymer chains may each independently be linear or branched.

Star polymer formations of the branched polymer according to the invention have been found to provide for excellent properties.

By "support moiety" is meant a moiety, such as an atom, molecule or core structure, to which is covalently attached the arms of the branched polymer. Accordingly, the support moiety functions to support the covalently attached arms.

To assist with describing the branched polymer, and in particular what is intended by the expressions "branched polymer" and "support moiety", reference may be made to general formula (A) below: 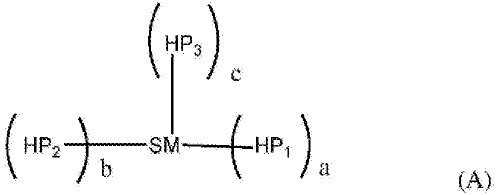 where SM represents the support moiety, HP₁ represents a cationic homopolymer chain, HP₂ represents a hydrophilic homopolymer chain, HP₃ represents a hydrophobic homopolymer chain, and a, b and c are each independently integers greater than or equal to 1, for example each independently being an integer ranging from 1 to 10. Where a, b or c are greater than 1 it will be appreciated that this will represent a situation where more than one relevant homopolymer chain is covalently coupled to the support moiety. One or more of the at least three homopolymer arms (HP₁, HP₂ and HP₃) may be covalently coupled to SM through a linking moiety.

With reference to general formula (A), the support moiety (SM) has each of the at least three homopolymer arms (HP₁, HP₂ and HP₃) covalently coupled thereto, and as such, SM may simplistically be viewed as a structural feature from which branching occurs.

The features of general formula (A) may provide for a variety of branched structures.

The branched polymer in accordance with the invention may have more of such structural features from which branching occurs. For example, the support moiety may be a linear macromolecule to which are covalently attached in pendant form a plurality of cationic homopolymer chains, a plurality of hydrophilic homopolymer chains and a plurality of hydrophobic homopolymer chains so as to form a polymer brush structure.

Accordingly, the branched polymer according to the invention may be described as comprising a structural feature of general formula (A).

Where the support moiety is an atom, it will generally be C, Si or N, In the case where the atom is C or Si, there may be a fourth polymer chain covalently coupled to the respective atom.

Where the support moiety is a molecule, there is no particular limitation concerning the nature of the moiety provided it can have the at least three homopolymer arms covalently coupled to it. In other words, the molecule must be at least tri-valent (i.e. have at least three points at which covalent attachment occurs). For example, the molecule can be selected from at least tri-valent forms of optionally substituted: alkyl, alkenyl, alkynyl, aryL carbocyclyL heterocyclyl, heteroaryl, alkyloxy, alkenyloxy, alkynyloxy, aryloxy, carbocyclyloxy, heterocyclyloxy, heteroaryloxy, alkylthio, alkenylthio, alkynylthio, arylthio, carbocyclylthio, heterocyclylthio, heteroarylthio, alkylalkenyl, alkylalkynyl, alkylaryl, alkylacyl, alkylcarbocyclyl, alkylheterocyclyl, alkylheteroaryl, alkyloxyalkyl, alkenyloxyalkyl, alkynyloxyalkyl, aryloxyalkyl, alkylacyloxy, alkyloxyacylalkyl, alkylcarbocyclyloxy, alkylheterocyclyloxy, alkylheteroaryloxy, alkylthioalkyl. alkenylthioalkyl, alkynylthioalkyl, arylthioalkyl, alkylacylthio, alkylcarbocyclylthio, alkylheterocyelylthio, alkylheteroarylthio, alkylalkenylalkyl, alkylalkynylalkyl, alkylarylalkyl, alkylacylalkyl, arylalkylaryl, arylalkenylaryl, arylalkynylaryl, arylacylaryl, arylacyl, aryicarbocyclyl, arylheterocyclyl, arylheteroaryl, alkenyloxyaryl, alkynyloxyaryl, aryloxyaryl, arylaeyloxy, arylcarbocyclyloxy, arylheterocyclyloxy, arylheteoaryloxy, alkylthioaryl, alkenylthioaryl, alkynylthioaryl, arylthioaryl, arylacylthio, arylcarbocyclylthio, arylheterocyclylthio, arylheteroarylthio, coordination complex, and a polymer chain, wherein where present the or each -CH₂- group in any alkyl chain may be replaced by a divalent group independently selected from -O-, -OP(O)₂-, -OP(O)₂O-, -S-,-S(O)-, -S(O)₂O-, -OS(O)₂O-, -N=N-, -OSi(OR^{a})₂O-, -Si(OR^{a})₂O-, -OB(OR^{a})O-, -B(OR^{a})O-, -NR^{a}-, -C(O)-, -C(O)O-, -OC(O)O-, -OC(O)NR^{a}- and -C(O)NR^{a}-, where the or each R^{a} may be independently selected from hydrogen, alkyl, alkenyl, alkynyl, aryl, carbocyclyl, heteroaryl, heterocyclyl, arylalkyl, and acyl, The or each R^{a} may also be independently selected from hydrogen, C₁₋₁₈alkyl, C₁₋₁₈alkenyl, C₁₋₁₈alkynyl, C₆₋₁₈aryl, C₃₋₁₈carbocyclyl, C₃₋₁₈heteroaryl, C₃₋₁₈heterocyclyl, and C₇₋₁₈arylalkyl,

Where the support moiety is a core structure, there is no particular limitation concerning the nature of the moiety provided it can have the at least three homopolymer arms covalently coupled to it. In other words, the core structure must have at least three points at which the covalent attachment occurs. For example, the core structure may be in the form of solid particulate material such as nano-particulate material, or polymer particles such as crosslinked polymer particles. In that case, the size of the core structure will generally range from about 5 angstroms to about 500 nm, for example from about 10 angstroms to about 100 nm. or from about 20 angstroms to about10 nm.

Examples of crosslinked polymer support moieties include those that contain the polymerised residues, or are formed through polymerisation, of one ore more multifunctional monomers selected from disulfide dimethacrylate, ethylene glycol di(meth)acrylate, triethylene glycol di(meth)acrylate, tetraethylene glycol di(meth)acrylate, 1,3-butylene glycol di(meth)acrylate, trimethylolpropane tri(meth)acrylate, 1,4-butanediol di(meth)acrylate, neopentyl glycol di(meth)acrylate, 1-6-hexanediol di(meth)acrylate, 1,6-hexanediol ethoxylated diacrylate (HEDA), pentaerythritol di(meth)acrylate, pentaerythritol tri(meth)acrylate, pentaerythritol tetra(meth)acrylate, glycerol di(meth)acrylate, glycerol allyloxy di(meth)acrylate, 1,1,1-tris(hydroxymethyl)ethane di(meth)acrylate, 1,1,1-tris(hydroxymethyl)ethanetri(meth)acrylate, 1,1,1-tris(hydroxymethyl)propane di(meth)acrylate, 1,1,1-tris(hydroxymethyl)propane tri(meth)acrylate, triallyl cyanurate, triallyl isocyanurate, triallyl trimellitate, diallyl phthalate, diallyl terephthalte, divinyl benzene, methylol (meth)acrylamide, triallylamine, oleyl maleate, glyceryl propoxy triacrylate, allyl methacrylate, methacrylic anhydride, methylenebis (meth) acrylamide, but-2-ene-1,4-diacrylate (BDA), bisphenol A ethoxylated diacrylate (BEDA), N,N'- bis(acryloyl) cystanimine (DSBAm) divinyl adipate (DVA), 4,4'-divinylhiphenyl (DVPB) and N,N'-methylenebisacrylamide (MBA).

Where the branched polymer comprises only one support moiety and the at least three homopolymer arms are linear, the branched polymer may be conveniently referred to as a star polymer or a mikto-arm star polymer.

When defining the support moiety it can also be convenient to refer to a compound or core structure from which the moiety is derived. For example, the support moiety may be derived from a compound or core structure having three or more functional groups that provide reactive sites through which the at least three homopolymer arms are to be covalently coupled. In that case, the support moiety may be derived from a compound or core structure having three or more functional groups selected from, for example, alcohol, halogen, thiol, carboxylic acid, amine, epoxide, isocyanate, and acid chloride.

Examples of compounds having three or more alcohol functional groups from which the support moiety may be derived include, but are not limited to, glycerol, pentaerythritol, dipentaerythritol, tripentaerythritol, 1,2,3-trihydroxyhexane, trimethylolpropane, myo-inisitol, glucose and its isomers (e.g. d-galactose, d-manose, d-fructose), maltose, sucrose, and manitol.

Examples of compounds having three or more functional (carboxylic, mercapto, halogen or isocyanate) groups from which the support moiety may be derived include, but are not limited to Biphenyl-3,4',5-tricarboxylic acid, cis,cis,-1,3,5-trimethylcyclohexane-1,3, 5-tricarboxylic acid, 1,2,4-benzenetricarboxylic acid, agaric acid, benzene-1,3,5-tricarboxylic acid, Methyl 1,1,2-ethanetncarboxylic acid, cyclohexane-1,24,5-tetracarboxylic acid, cyclopentane-1,2,3,4-tetracarboxytic acid, bicycle[2.2.2]oct-7-ene-2,3,5,6-tetracarboxylic acid, pentaerythritol tetrakis(3-mercaptopriopionate), 1,2,3-trichloropropane, 1,3,3-trichlorobutane, 1,2,3-trichlorobutane, lysine triisocyanate, and triphenylmethane-4,4',4'-triisocyanate,

Examples of compounds having three or more amine functional groups from which the support moiety may be derived include, but are not limited to poly(ethylene imine), 1,2,3-triamino-2-(aminomethyl)propane, PAMAM dendrimer, butane-1,2,3,4-tetraamine, and pentane-1,2,3,3-tetraamine.

The at least three homopolymer chains are each (i.e. separately) covalently coupled to the support moiety. Each homopolymer chain may be covalently coupled directly or indirectly to the support moiety.

By being "directly" covalently coupled is meant that there is only a covalent bond between the homopolymer chain and the support moiety.

By being "indirectly" covalently coupled is meant that there is located between the homopolymer chain and the support moiety one or more covalently bonded atoms or molecules.

Where the homopolymer chains are indirectly covalently coupled to the support moiety, it may be convenient to refer to the homopolymer chains as being covalent coupled to the support moiety through a linking moiety.

In one embodiment, at least one of the at least three homopolymer chains is covalently coupled to the support moiety through a linking moiety.

In another embodiment, each of the at least three homopolymer chains are each covalently coupled to the support moiety through a linking moiety.

There is no particular limitation concerning the nature of such a linking moiety provided it can function to couple a homopolymer chain to the support moiety. The linkage moiety will of course not be another polymer chain as this would result in formation of an overall copolymer chain being coupled to the support moiety.

Examples of suitable linking moieties include a divalent form of optionally substituted: oxy (-O-), disulfide (-S-S-), alkyl, alkenyl alkynyl, aryl, acyl (including -C(O)-), carbocyclyl, heterocyclyl, heteroaryL alkyloxy, alkenyloxy, alkynyloxy, aryloxy, acyloxy, carbocyclyloxy, heterocyclyloxy, heteroaryloxy, alkylthio, alkenylthio, alkynylthio, arylthio, acylthio, carbocyclylthio, heterocyclylthio, heteroarylthio, alkylalkenyl, alkylalkynyl, alkylaryl, alkylacyl, alkylcarbocyclyl, alkylheterocyclyl, alkylheteroaryl, alkyloxyalkyl, alkenyloxyalkyl, alkynyloxyallkyl, aryloxyalkyl, alkylacyloxy, alkyloxyacylalkyl, alkylcarbocyclyloxy, alkylheterocyclyloxy, alkylheteroaryloxy, alkylthioalkyl, alkenylthioalkyl alkynylthioalkyl, arylthioalkyl, alkylacylthio, alkylcarbocyclylthio, alkylheterocyclylthio, alkylheteroarylthio, alkylalkenylalkyl, alkylalkynylalkyl, alkylarylalkyl, alkylacylalkyl, arylalkylaryl, arylalkenylaryl, arylalkynylaryl, arylacylaryl, arylacyl, arylcarbocyclyl, arylheterocyclyl, arylheteroaryl, alkenyloxyaryl alkynyloxyaryl, aryloxyaryl, arylacyloxy, arylcarbocyclyloxy, arylheterocyclyloxy, arylheteroaryloxy, alkylthioaryl, alkenylthioaryl, alkynylthioaryl, arylthioaryl, arylacylthio, arylcarbocyclylthio, arylheterocyclylthio, and arylheteroarylthio, wherein where present the or each -CH₂- group in any alkyl chain may be replaced by a divalent group independently selected from -(O)-, -OP(O)₂-, -OP(O)₂O-, -S-, -S(O)-, -S(O)₂O-. -OS(O)₂O-. -N=N-, -OSi(OR^{a})₂O-, -Si(OR^{a})₂O-, -OB(OR^{a})O-, -B(OR^{a})O-, -NR^{a}-, -C(O)-, -C(O)O-, -OC(O)O-, -OC(O)NR^{a}- and -C(O)NR^{a}-, where the or each R^{a} may be independently selected from hydrogen, alkyl, alkenyl, alkynyl, aryl, carbocyclyl, heteroaryl, heterocyclyl, arylalkyl, and acyl. The or each R^{a} may also be independently selected from hydrogen, C₁₋₁₈alkyl, C₁₋₁₈alkenyl, C₁₋₁₈alkynyl, C₆₋₁₈aryl C₃₋₁₈carbocyclyl. C₃₋₁₈heteroaryl, C₃₋₁₈heterocyclyl, and C₇₋₁₈arylalkyl.

Reference herein to groups containing two or more subgroups (e.g. [group A][group B]), is not intended to be limited to the order in which the subgroups are presented. Thus, two subgroups defined as [group A][group B] (e.g. alkylaryl) is intended to also be a reference to two subgroups defined as [group B][group A] (e.g. arylalkyl).

The linking moiety may be or comprise a degradable functional group.

In one embodiment, one or more of the at least three homopolymer chains is covalently coupled to the support moiety through a degradable functional group such that upon the degradable functional group undergoing degradation the covalent couple is cleaved and the homopolymer chain is severed from the support moiety.

Each of the at least three homopolymer chains may be covalently coupled to the support moiety through a degradable functional group.

In another embodiment, the support moiety has a molecular structure comprising one or more degradable functional groups such that upon the degradable functional group undergoing degradation one or more covalent bonds in that molecular structure are cleaved and at least one of the at least three the homopolymer chains is released (i.e. becomes free) from the support moiety.

By a functional group being "degradable" is meant that it has a molecular structure that is susceptible to break down (i.e. undergoing bond cleavage) via a chemical reaction upon being exposed to a degrading environment.

In one embodiment, the degradable functional group is a biodegradable functional group.

By a functional group being "biodegradable" is meant that it has a molecular structure that is susceptible to break down (i.e. undergoing bond cleavage) via a chemical reaction upon being exposed to a biological environment (e.g. within a subject or in contact with biological material such as blood, tissue etc). Biodegradation may occur invitro or invivo. Such chemical decomposition or biodegradation may be via hydrolysis, oxidation or reduction. Accordingly, the biodegradable functional groups will generally be susceptible to undergoing hydrolytic, oxidative or reductive cleavage. The rate of biodegradation may vary depending on a number of extrinsic or intrinsic factors (e. g. light, heat, radiation, pH, enzymatic or nonenzymatic mediation, etc.).

The degradable functional groups are positioned such that when they undergo degradation one or more of the at least three homopolymer chains is released from (i.e. is no longer covalently attached to) the support moiety.

Where the support moiety has a molecular structure that comprises degradable functional groups, degradation of those degradable functional groups results in the support moiety structure breaking down, which in turn results in a homopolymer chain being released from (i.e. is no longer covalently attached to) the support moiety.

Those skilled in the art will appreciate the type of functional groups that are susceptible to undergoing degradation and biodegradation. Such functional groups may include, for example, ester, anhydride, carbonate, peroxide, peroxyester, phosphate, thioester, urea, thiourethane, ether, disulfide, carbamate (urethane) and boronate ester.

In one embodiment, the biodegradable functional groups are selected from ester, anhydride, carbonate, peroxide, peroxyester, phosphate, thioester, urea, thiourethane, ether, disulfide, carbamate (urethane) and boronate ester.

In another embodiment, the linking moiety is biodegradable through one or more functional groups selected from ester, anhydride, carbonate, peroxide, peroxyester, phosphate, thioester, urea, thiourethane, ether, disulfide, carbamate (urethane) and boronate ester.

Degradation of a biodegradable functional group may be facilitated in the presence of an acid, a base, an enzyme and/or another endogenous biological compound that can catalyze or at least assist in the bond cleavage process. For example, an ester may be hydrolytically cleaved to produce a carboxylic acid group and an alcohol group, an amide may be hydrolytically cleaved to produce a carboxylic acid group and an amine group, and a disulfide may be reductively cleaved to produce thiol groups.

Degradation may occur in a biological fluid such as blood, plasma, serum, urine, saliva, milk, seminal fluid, vaginal fluid, synovial fluid, lymph fluid, amniotic fluid, sweat, and tears; as well as an aqueous solution produced by a plant, including, for example, exudates and guttation fluid, xylem, phloem, resin, and nectar.

Degradation may also occur in a cell or in cellular components such as endosomes and cytoplasm.

Biodegradable functional groups may be selected such that they can undergo degradation upon being exposed to a particular biological environment. For example, a redox potential gradient exists between extracellular and intracellular environments in normal and pathophysiological states. Disulfide bonds as a biodegradable functional group may be readily reduced in the reducing intracellular environment, while remaining intact in the oxidizing extracellular space. The intracellular reduction of the disulfide bond is typically executed by small redox molecules such as glutathione (GSH) and thioredoxin, either alone or with the help of enzymatic machinery.

Where two or more degradable functional groups are used, depending on the nature of these functional groups and the degradation environment, it may be that only one of the functional groups actually promotes the desired bond cleavage. For example, the degradable functional groups may include both ester and disulfide functional groups. In a reductive environment, it may be that only the disulfide functional group will undergo degradation. In a hydrolytic environment it may be that only the ester functional group will undergo degradation. In a reductive and hydrolytic environment it may be that both the disulfide and ester functional groups will undergo degradation.

By a "cationic" homopolymer chain is meant a homopolymer chain that presents or is capable of presenting a net positive charge. In one embodiment, the cationic homopolymer chain presents a net positive charge.

By a "hydrophilic" homopolymer chain is meant a homopolymer chain that presents net hydrophilic character. The hydrophilic homopolymer chain will generally not present or be capable of presenting a net negative or net positive charge. The hydrophilic homopolymer chain may therefore be described as a hydrophilic homopolymer chain that does not present, or is not capable of presenting, a net negative or net positive charge. In other words, the hydrophilic homopolymer chain is not intended to be a cationic homopolymer chain as described herein (i.e. it does not present or is not capable of presenting a net positive charge). Accordingly, the hydrophilic homopolymer chain may also be described as a non-cationic hydrophilic homopolymer chain.

In one embodiment, the hydrophilic homopolymer chain is a neutral hydrophilic homopolymer chain (i.e. it does not present or is not capable of presenting a charge). In that case, the hydrophilic homopolymer chain may be described as being a non-ionisable hydrophilic homopolymer chain.

By a "hydrophobic" homopolymer chain is meant a homopolymer chain that presents net hydrophobic character. The hydrophobic homopolymer chain will generally not present or be capable of presenting a net negative or positive charge. The hydrophobic homopolymer chain may therefore be described as a hydrophobic homopolymer chain that does not present, or is not capable of presenting, a net negative or net positive charge. In other words, the hydrophobic homopolymer chain is not intended to be a cationic homopolymer chain as defined herein (i.e. it does not present or is not capable of presenting a net positive charge). Accordingly, the hydrophobic homopolymer chain may also be described as a non-cationic hydrophobic homopolymer chain.

In one embodiment, the hydrophobic homopolymer chain is a neutral hydrophobic homopolymer chain (i.e. it does not present or is not capable of presenting a charge). In that case, the hydrophobic homopolymer chain may be described as being a non-ionisable hydrophobic homopolymer chain.

Further detail regarding what is meant by the expressions "cationic", "hydrophilic" and "hydrophobic" homopolymer chains is presented below.

The cationic, hydrophilic and hydrophobic homopolymer chains will cach comprise the polymerised residues of a plurality of monomer units. Further detail concerning the monomers that may be used to form these homopolymer chains is presented below.

A cationic homopolymer chain in accordance with the invention will generally comprise from about 5 to about 250, or about 10 to about 200, or about 30 to about 150 monomer residue units.

The cationic homopolymer chain may exhibit hydrophilic character in the sense that it is soluble or miscible in aqueous media.

In one embodiment, the branched polymer does not comprise polymerised monomer residue units bearing or that are capable of bearing negative charge. In other words, in one embodiment the branched polymer is not an ampholytic branched polymer.

Reference herein to "positive" or "negative" charge associated with, for example, a cationic homopolymer chain or nucleic acid molecule, respectively, is intended to mean that the cationic homopolymer chain or nucleic acid molecule has one or more functional groups or moieties that present, or are intended to and are capable of presenting, a positive or negative charge, respectively.

Accordingly, such a functional group or moiety may inherently bear that charge, or it may be capable of being converted into a charged state, for example through addition or removal of an electrophile. In other words, in the case of a positive charge, a functional group or moiety may have an inherent charge such as a quaternary ammonium functional group or moiety, or a functional group or moiety *per se* may be neutral, yet be chargeable to form a cation through, for example, pH dependent formation of a tertiary ammonium cation, or quaternerisation of a tertiary amine group. In the case of negative charge, a functional group or moiety may, for example, comprise an organic acid salt that provides for the negative charge, or a functional group or moiety may comprise an organic acid which may be neutral, yet be chargeable to form an anion through, for example, pH dependent removal of an acidic proton.

In one embodiment, a cationic homopolymer chain of the branched polymer may be prepared using monomer that contains a functional group or moiety that is in a neutral state at the time of polymerisation and the so form polymer chain can subsequently converted into a positively charged state. For example, the monomer may comprise a tertiary amine functional group, which upon being polymerised to form the cationic homopolymer chain is subsequently quaternarised into a positively charged state.

In another embodiment, the cationic homopolymer chain of the branched polymer is in a positively charged state. In other words, the cationic homopolymer chain is a positively charged cationic homopolymer chain.

Those skilled in the art will appreciate that in a charged state, a cation *per se* associated with a cationic homopolymer chain, or an anion *per se* associated with, for example, a nucleic acid molecule, will have a suitable counter ion associated with it.

Where a branched polymer according to the invention is used in complex formation with a nucleic acid molecule, it will be appreciated that a cationic homopolymer chain, individually or collectively (if more than one is present), will comprise sufficient positive charge sites to promote complexation with the nucleic acid molecule.

In one embodiment, the cationic homopolymer chain in accordance with the invention may comprise from about 5 to about 250, or about 10 to about 200, or about 30 to about 150 monomer residue units that each comprise a positive charge.

A hydrophilic homopolymer chain in accordance with the invention will generally comprise from about 5 to about 200, or about 10 to about 150, or about 20 to about 100 monomer residue units.

A hydrophobic homopolymer chain in accordance with the invention will generally comprise from about 10 to about 200, or about 15 to about 150, or about 30 to about 100 monomer residue units.

Terms such as hydrophilic and hydrophobic are generally used in the art to convey interactions between one component relative to another (e.g. attractive or repulsive interactions, or solubility characteristics) and not to quantitatively define properties of a particular component relative to another.

For example, a hydrophilic component is more likely to be wetted or solvated by an aqueous medium such as water, whereas a hydrophobic component is less likely to be wetted or solvated by an aqueous medium such as water.

In the context of the present invention, a hydrophilic homopolymer chain is intended to mean a homopolymer chain that exhibits solubility or miscibility in an aqueous medium, including biological fluids such as blood, plasma, serum, urine, saliva, milk, seminal fluid, vaginal fluid, synovial fluid, lymph fluid, amniotic fluid, sweat, and tears; as well as an aqueous solution produced by a plant, including, for example, exudates and guttation fluid, xylem, phloem, resin, and nectar.

In contrast, a hydrophobic homopolymer chain is intended to mean homopolymer chain that exhibits little or no solubility or miscibility in an aqueous medium, including biological fluids such as blood, plasma, serum, urine, saliva, milk, seminal fluid, vaginal fluid, synovial fluid, lymph fluid, amniotic fluid, sweat, and tears; as well as an aqueous solution produced by a plant, including, for example, exudates and guttation fluid, xylem, phloem, resin, and nectar.

The cationic, hydrophilic or hydrophobic character of a given homopolymer chain can be readily determined by a person skilled in the art through simple assessment of (a) the molecular composition of the homopolymer chain, and/or (b) the solubility or miscibility (or lack thereof) of the homopolymer chain in an aqueous medium.

The hydrophilic homopolymer chain will generally be selected such that the branched polymer is rendered soluble or miscible in aqueous media.

In one embodiment, the hydrophilic homopolymer chain may comprise from about 5 to about 200. or about 10 to about 150, or about 20 to about 100 hydrophilic monomer residue units.

In one embodiment, the hydrophobic homopolymer chain may comprise from about 10 to about 200. or about 15 to about 150, or about 30 to about 100 hydrophobic monomer residue units

Each of the homopolymer chain may be derived from (or comprise a polymerised residue of) monomer comprising groups having a branched structure.

The cationic homopolymer chain may be derived from (or comprise a polymerised residue of) one or more monomers selected from N,N-dimethyaminoethyl methacrylate, N,N-diethyaminoethyl methacrylate, N,N-dimethylaminoethyl acrylate, N,N-diethylaminoethyl acrylate, 2-aminoethyl methacrylate hydrochloride, N-[3-(N,N-dimethylamino)propyl]methacrylamide, N-(3-aminopropyl)methacrylamide hydrochloride, N-[3-(N,N-dimethylamino)propyl] acrylamide. N-[2-(N,N-dimethylamino)ethyl]methacrylamide, 2-N-morpholinoethyl acrylate, 2-N-morpholinoethyl methacrylate, 2-(N,N-dimethylamino)ethyl acrylate, 2-(N,N-dimethylamino)ethyl methacrylate, 2-(N,N-diethylamino)ethyl methacrylate, 2-acryloxyyethyltrimethylammonium chloride, mthacrylamidopropyltrimethylammonium chloride, 2-(tert-butylamino)ethyl methacrylate, allyldimethylammonium chloride, 2-(dethylamino)ethylstyrene, 2-vinylpyridine, and 4-vinylpyridine.

The hydrophilic homopolymer chain may be derived from (or comprise a polymerised residue of) one or more monomers selected from acrylic acid, methacrylic acid, hydroxyethyl methacrylate, hydroxypropyl methacrylate, oligo(alkylene glycol)methyl ether (meth)acrylate (OAG(M)A), oligo(ethylene glycol) (meth)acrylate (OEG(M)A), acrylamide and methacrylamide, hydroxyethyl acrylate, N-methylacrylamide, N,N-dimethylacrylamide, N,N-dimethylaminoethyl methacrylate, N,N-dimethylaminopropyl methacrylamide, N-hydroxypropyl methacrylamide, 4-acryloylmorpholine, 2-acrylamido-2-methy)-1-propanesulfonic acid, phosphorylcholine methacrylate and N-vinyl pyrolidone.

The hydrophobic homopolymer chain may be derived from (or comprise a polymerised residue of) one or more monomers selected from styrene, alpha-methyl styrene, butyl acrylate, butyl methacrylate, amyl methacrylate, hexyl methacrylate, lauryl methacrylate, stearyl methacrylate, ethyl hexyl methacrylate, crotyl methacrylate, cinnamyl methacrylate, oleyl methacrylate, ricinoleyl methacrylate, cholesteryl methacrylates, cholesteryl acrylate, vinyl butyrate, vinyl tert-butyrate, vinyl stearate and vinyl laurate.

Examples of branched polymers according to the invention may be illustrated with reference to general formulae (A) below: 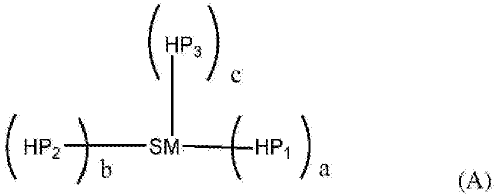 where SM represents the support moiety, HP₁ represents a cationic homopolymer chain derived from (i.e. is a polymerised form of) N,N-dimethyaminoethyl methacrylate, N.N-diethyaminoethyl methacrylate, N,N-dimethylaminoethyl acrylate, N,N-diethylaminoethyl acrylate, 2-aminoethyl methacrylate hydrochloride, N-[3-(N,N-dimethylamino)propyl]methacrylamide, N-(3-aminopropyl)methacrylamide hydrochloride. N-[3-(N,N-dimethylamino)propyl]acrylamide, N-[2-(N,N-dimethylamino)ethyl]methacrylamide, 2-N-morpholinoethyl acrylate, 2-N-morpholinoethyl methacrylate, 2-(N,N-dimethylamino)ethyl acrylate, 2-(N,N-dimethylamino)ethyl methacrylate, 2-(N,N-diethylamino)ethyl methacrylate, 2-acryloxyyethyltrimethylammonium chloride, mthacrylamidodpropyltrimethylammonium chloride, 2-(tert-butylamino)ethyl methacrylate, allyldimethylammonium chloride, 2-(dethylamino)ethylstyrene, 2-vinylpyridine, or 4-vinylpyridine. HP₂ represents a hydrophilic homopolymer chain derived from (i.e. is a polymerised from of) acrylic acid, methacrylic acid, hydroxyethyl methacrylate, hydroxypropyl methacrylate, oligo(alkylene glycol)methyl ether (meth)acrylate (OAG(M)A), oligo(ethylene glycol) (meth)acrylate (OEG(M)A), acrylamide and methacrylamide, hydroxyethyl acrylate, N-methylacrylamide, N,N-dimethylacrylamide, N,N-dimethylaminoethyl methacrylate, N,N-dimethylaminopropyl methacrylamide, N-hydroxypropyl methacrylamide, 4-acryloylmorpholine, 2-acrylamido-2-methyl-1-propanesulfonic acid, phosphorylcholine methacrylate or N-vinyl pyrolidone, HP₃ represents a hydrophobic homopolymer chain derived from (i.e. is a polymerised from of) styrene, alpha-methyl styrene, butyl acrylate, butyl methacrylate, amyl methacrylate, hexyl methacrylate, lauryl methacrylate, stearyl methacrylate, ethyl hexyl methacrylate, crotyl methacrylate, cinnamyl methacrylate, oleyl methacrylate, ricinoleyl methacrylate, cholesteryl methacrylates, cholesteryl acrylate, vinyl butyrate, vinyl tert-butyrate, vinyl stearate or vinyl laurate, and a, b and c are each independently integers ranging from 1 to 50, or from 1 to 30, or from 1 to 20, or from 1 to 15. or from 1 to 10, or from 1 to 5. Where a, b or c are greater than I it will be appreciated that this will represent a situation where more than one relevant homopolymer chain is covalently coupled to the support moiety. One or more of the at least three homopolymer arms (HP₁ HP₂ and HP₃) may be covalently coupled to SM through a linking moiety.

In one embodiment, the branched polymer further comprises a targeting ligand, a bioactive and/or an imaging agent. In that case, a targeting ligand, bioactive or an imaging agent will generally be covalently coupled to the branched polymer. A targeting ligand, bioactive or an imaging agent may be covalently coupled to the support moiety, one or more of the at least three homopolymer chains of the branched polymer, or a combination thereof. A combination of two or more of a targeting ligand, bioactive and/or imaging agent is also contemplated.

Examples of suitable targeting ligands that may be coupled to the branched polymer include sugars and oligosaccharides derived from those sugars, peptides, proteins, aptamers, and cholesterol. Examples of suitable sugars include galactose, mannose, and glucosamine. Examples of suitable peptides include bobesin, lutanizing hormone releasing peptide, cell penetrating peptides (CPP's), GALA peptide, influenza-derived fusogeneic peptides, RGD peptide, poly(arginine), poly(lycine), penetratin, tat-peptide, and transportan. Other ligands such as folic acid that can target cancer cells may also be coupled to the branched polymer. Examples of bioactives that may be coupled to the branched polymer include active organic compounds, proteins or antibodies (or fragments thereof), and peptides, Examples of suitable proteins include transferring prolamine, and antibodies such as anti-EGFR antibody and anti-K-ras antibody. Examples of suitable organic compounds include chemotherapeutic agents like doxorubicin, paclitaxel, camptothecins and palatinates.

Examples of suitable imaging agents that may be coupled to the branched polymer include Polyfluor™ (Methacryloxyethyl thiocarbamoyl rhodamine B), Alexa Fluor 568, and BOPIDY dye.

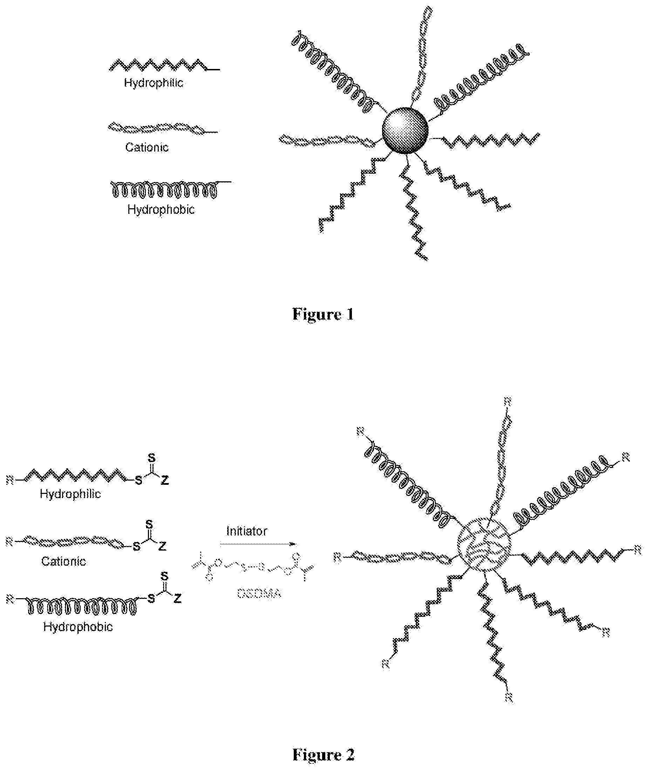

To further illustrate the nature of branched polymers in accordance with the invention, reference is made to Figure 1 in which 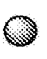 represents the support moiety, - represents a general covalent bond, 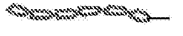 represents a cationic homopolymer chain. 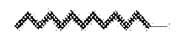 represents a hydrophilic homopolymer chain, and 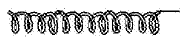 represents a hydrophobic homopolymer chain. One or more of the homopolymer chains may be covalently coupled to the support moiety through a linking moiety (not shown). The linking moiety may be, or comprise, a biodegradable functional group.

The branched polymers according to the invention may be prepared by any suitable means.

In one embodiment, the process of preparing the branched polymer comprises the polymerisation of ethylenically unsaturated monomers. Polymerisation of the ethylenically unsaturated monomers is preferably conducted using a living polymerisation technique.

Living polymerisation is generally considered in the art to be a form of chain polymerisation in which irreversible chain termination is substantially absent. An important feature of living polymerisation is that polymer chains will continue to grow while monomer and reaction conditions to support polymerisation are provided. Polymer chains prepared by living polymerisation can advantageously exhibit a well defined molecular architecture, a predetermined molecular weight and narrow molecular weight distribution or low polydispersity.

Examples of living polymerisation include ionic polymerisation and controlled radical polymerisation (CRP). Examples of CRP include, but are not limited to, iniferter polymerisation, stable free radical mediated polymerisation (SFRP), atom transfer radical polymerisation (ATRP), and reversible addition fragmentation chain transfer (RAFT) polymerisation.

Equipment, conditions, and reagents for performing living polymerisation are well known to those skilled in the art.

Where ethylenically unsaturated monomers are to be polymerised by a living polymerisation technique, it will generally be necessary to make use of a so-called living polymerisation agent. By "living polymerisation agent" is meant a compound that can participate in and control or mediate the living polymerisation of one or more ethylenicalty unsaturated monomers so as to form a living polymer chain (i.e. a polymer chain that has been formed according to a living polymerisation technique).

Living polymerisation agents include, but are not limited to, those which promote a living polymerisation technique selected from ionic polymerisation and CRP.

In one embodiment of the invention, the branched polymer is prepared using ionic polymerisation.

In one embodiment of the invention, the branched polymer is prepared using CRP.

In a further embodiment of the invention, the branched polymer is prepared using iniferter polymerisation.

In another embodiment of the invention, the branched polymer is prepared using SFRP.

In a further embodiment of the invention, the branched polymer is prepared using ATRP.

In yet a further embodiment of the invention, the branched polymer is prepared using RAFT polymerisation.

A polymer formed by RAFT polymerisation may conveniently be referred to as a RAFT polymer. By virtue of the mechanism of polymerisation, such polymers will comprise residue of the RAFT agent that facilitated polymerisation of the monomer.

RAFT agents suitable for use in accordance with the invention comprise a thiocarbonylthio group (which is a divalent moiety represented by: -C(S)S-). RAFT polymerisation and RAFT agents are described in numerous publications such as WO 98/01478, Moad G.; Rizzardo, E; Thang S, H. Polymer 2008, 49, 1079-1131 and Aust. J. Chem., 2005, 58, 379-410; Aust. J. Chem., 2006, 59, 669-692; and Aust. J. Chem., 2009, 62. Suitable RAFT agents for use in preparing the branched polymers include xanthate, dithioester, dithiocarbamate and trithiocarbonate compounds.

RAFT agents suitable for use in accordance with the invention also include those represented by general formula (1) or (II): 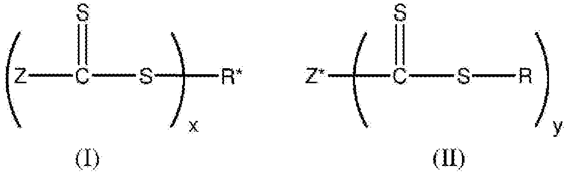 where Z and R are groups, and R* and Z* are x-valent and y-valent groups, respectively, that are independently selected such that the agent can function as a RAFT agent in the polymerisation of one or more ethylenically unsaturated monomers; x is an integer ≥ 1; and y is an integer ≥ 2.

In one embodiment, x is an integer ≥ 3; and y is an integer ≥ 3. In that case, R* and Z* may represent a support moiety (SM).

In order to function as a RAFT agent in the polymerisation of one or more ethylenically unsaturated monomers, those skilled in the art will appreciate that R and R* will typically be an optionally substituted organic group that function as a free radical leaving group under the polymerisation conditions employed and yet, as a free radical leaving group, retain the ability to reinitiate polymerisation. Those skilled in the art will also appreciate that Z and Z* will typically he an optionally substituted organic group that function to give a suitably high reactivity of the C=S moiety in the RAFT agent towards free radical addition without slowing the rate of fragmentation of the RAFT-adduct radical to the extent that polymerisation is unduly retarded.

In formula (1), R* is a x-valent group, with x being an integer ≥ 1. Accordingly, R* may be mono-valent, di-valent, tri-valent or of higher valency. For example. R* may be a C₂₀ alkyl chain, with the remainder of the RAFT agent depicted in formula (I) presented as multiple substituent groups pendant from the chain. Generally, x will be an integer ranging from 1 to about 20, for example from about 2 to about 10, or from 1 to about 5. In one embodiment x = 2.

Similarly, in formula (II), Z* is a y-valent group, with y being an integer ≥ 2. Accordingly, Z* may be di-valent, tri-valent or of higher valency. Generally, y will be an integer ranging from 2 to about 20, for example from about 2 to about 10, or from 2 to about 5.

Examples of R in RAFT agents that can be used in accordance with the invention include optionally substituted, and in the case of R* in RAFT agents that can be used in accordance with the invention include a x-valent form of optionally substituted: alkyl, alkenyl, alkynyl, aryl, acyl, carbocyclyl, heterocyclyl, heteroaryl, alkylthio, alkenylthio, alkynylthio, arylthio, acylthio, carbocyclylthio, heterocyclylthio, heteroarylthio, alkylalkenyl, alkylalkynyl, alkylaryl, alkylacyl, alkylcarbocyclyl, alkylheterocyclyl, alkylheteroaryl, alkyloxyalkyl, alkenyloxyalkyl, alkynyloxyalkyl, aryloxyalkyl, alkylacyloxy, alkylcarbocyclyloxy, alkylheterocyclyloxy, alkylheteroaryloxy, alkylthioalkyl, alkenylthioalkyl, alkynylthioalkyl, arylthioalkyl, alkylacylthio, alkylcarbocyclylthio, alkylheterocyclylthio, alkylheteroarylthio, alkylalkenylalkyl, alkylalkynylalkyl, alkylarylalkyl, alkylacylalkyl, arylalkylaryl, arylalkenylaryl, arylalkynylaryl, arylacylaryl, arylacyl, arylcarbocyclyl, arylheterocyclyl, arylheteroaryl, alkenyloxyaryl, alkynyloxyaryl, aryloxyaryl, alkylthioaryl, alkenylthioaryl, alkynylthioaryl, arylthioaryl, arylacylthio, arylcarbocyclylthio, arylheterocyclylthio, arylheteroarylthio, and a polymer chain.

For avoidance of any doubt reference herein to "optionally substituted:" alkyl, alkenyl, ... etc, is intended to mean each group such as alkyl and alkenyl is optionally substituted.

Examples of R in RAFT agents that can be used in accordance with the invention also include optionally substituted, and in the case of R* in RAFT agents that can be used in accordance with the invention also include an x-valent form of optionally substituted: alkyl; saturated, unsaturated or aromatic carbocyclic or heterocyclic ring; alkylthio; dialkylamino: an organometallic species; and a polymer chain.

Living polymerisation agents that comprise a polymer chain are commonly referred to in the art as "macro" living polymerisation agents. Such "macro" living polymerisation agents may conveniently be prepared by polymerising ethylenically unsaturated monomer under the control of a given living polymerisation agent.

In one embodiment, the at least three homopolymer chains are formed by polymerising ethylenically unsaturated monomer under the control of a living polymerisation agent, for example a RAFT agent.

Examples of Z in RAFT agents that can be used in accordance with the invention include optionally substituted, and in the case of Z* in RAFT agents that can be used in accordance with the invention include a y-valent form of optionally substituted: F, Cl, Br, I, alkyl, aryl, acyl, amino, carbocyclyl, heterocyclyl, heteroaryl, alkyloxy, aryloxy, acyloxy, acylamino. carbocyclyloxy, heterocyclyloxy, heteroaryloxy, alkylthio, arylthio, acylthio, carbocyclylthio, heterocyclylthio, heteroarylthio, alkylaryl, alkylacyl, alkylcarbocyclyl, alkylheterocyclyl, alkylheteroaryl, alkyloxyalkyl, aryloxyalkyl, alkylacyloxy, alkylcarbocyclyloxy, alkylheterocyclyloxy, alkylheteroaryloxy, alkylthioalkyl, arylthioalkyl, alkylacylthio, alkylcarbocyclylthio, alkylheterocyclylthio, alkylheteroarylthio, alkylarylalkyl, alkylacylalkyl, arylalkylaryl, arylacylaryl, arylacyl, arylcarbocyclyl, arylheterocyclyl, arylheteroaryl, aryloxyaryl, arylacyloxy, arylcarbocyclyloxy, arylheterocyclyloxy, arylheteroaryloxy, alkylthioaryl, arylthioaryl, arylacylthio, arylcarbocyclylthio, arylheterocyclylthio, arylheteroarylthio, dialkyloxy-, diheterocyclyloxy- or diaryloxy- phosphinyl, dialkyl-, diheterocyclyl- or diaryl-phosphinyl, cyano (i.e. -CN), and -S-R, where R is as defined in respect of formula (II).

In one embodiment, a RAFT agent tat can be used in accordance with the invention is a trithiocarbonate RAFT agent and Z or Z* is an optionally substituted alkylthio group. MacroRAFT agents suitable for use in accordance with the invention may be obtained commercially, for example see those described in the SigmaAldrich catalogue (www.sigmaaldrich.com).

Other RAFT agents that can be used in accordance with the invention include those described in WO2010/083569 and Benaglia et al. Macromolecules. (42), 9384-9386, 2009.

In the lists herein defining groups from which Z, Z*, R and R* may be selected, each alkyl, alkenyl, alkynyl, aryl, carbocyclyl, heteroaryl, heterocyclyl, and polymer chain moiety may be optionally substituted.

In the lists herein defining groups from which Z, Z*, R and R* may be selected, where a given Z, Z*, R or R* contains two or more subgroups (e.g. [group A][group B]), the order of the subgroups is not intended to be limited to the order in which they are presented (e.g. alkylaryl may also be considered as a reference to arylalkyl).

The Z, Z*, R or R* may be branched and/or optionally substituted. Where the Z, Z*, R or R* comprises an optionally substituted alkyl moiety, an optional substituent includes where a -CH₂- group in the alkyl chain is replaced by a group selected from -O-, -S-, -NR^{a}-, -C(O)- (i.e. carbonyl), -C(O)O- (i.e. ester), and -C(O)NR^{a}- (i.e. amide), where R^{a} may be selected from hydrogen, alkyl, alkenyl, alkynyl, aryl, carbocyclyl, heteroaryl, heterocyclyl, arylalkyl, and acyl.

Reference herein to a x-valent, y-valent, multi-valent or di-valent "form of...." is intended to mean that the specified group is a x-valent, y-valent, multi-valent or di-valent radical, respectively. For example, where x or y is 2, the specified group is intended to be a divalent radical. Those skilled in the art will appreciate how to apply this rationale in providing for higher valent forms.

Preparation of the branched polymers will generally involve the polymerisation of ethylenically unsaturated monomer.

Suitable examples of ethylenically unsaturated monomers that may be used to prepare the branched polymers include those of formula (III): 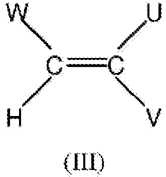
where U and W are independently selected from -CO₂H, -CO₂R¹, -COR¹, -CSR¹,-CSOR¹, -COSR¹, -CONH₂, -CONHR¹, -CONR¹₂, hydrogen, halogen and optionally substituted C₁-C₄ alkyl or U and W form together a lactone, anhydride or imide ring that may itself be optionally substituted, where the optional substituents are independently selected from hydroxy, -CO₂H, -CO₂R¹, -COR¹, -CSR¹, -CSOR¹, -COSR¹, -CN, -CONH₂, -CONHR¹, -CONR¹₂, -OR¹, -SR¹, -O₂CR¹, -SCOR¹, and - OCSR¹;
V is selected from hydrogen, R¹, -CO₂H, -CO₂R¹, -COR¹, -CSR¹, -CSOR¹,-COSR¹, -CONH₂, -CONHR¹, -CONR¹₂, -OR¹, -SR¹, -OCR¹, -SCOR¹, and - OCSR¹;
where the or each R¹ is independently selected from optionally substituted alkyl, optionally substituted alkenyl, optionally substituted alkynyl, optionally substituted aryl, optionally substituted heteroaryl, optionally substituted carbocyclyl, optionally substituted heterocyclyl, optionally substituted arylalkyl, optionally substituted heteroarylalkyl, optionally substituted alkylaryl, optionally substituted alkylheteroaryl, and an optionally substituted polymer chain.

Specific examples of monomers of formula (III) include those outlined in one or more of WO 2010/083569, WO 98/01478, Moad G.; Rizzardo, E; Thang S, H. Polymer 2008, 49, 1079-1131 and Aust. J. Chem., 2005, 58, 379-410; Aust. J. Chem., 2006, 59, 669-692; Aust. J. Chem., 2009, 62, 1402-1472, Greenlee, R. Z., in Polymer Handbook 3rd edition (Brandup. J, and Immergut. E. H. Eds) Wiley: New York, 1989, p II/53 and Benaglia et al, Macromolecules, (42), 9384-9386, 2009.

When discussing the types of monomers that are used to prepare the branched polymer, it is convenient to refer to the monomers as being hydrophilic, hydrophobic or cationic in character. By being hydrophilic, hydrophobic or cationic "in character" in this context is meant that upon polymerisation such monomers respectively give rise to the hydrophilic, hydrophobic and cationic homopolymer chains. For example, a hydrophilic homopolymer chain will be prepared by polymerising hydrophilic monomer.

As a guide only, examples of hydrophilic ethylenically unsaturated monomers include, but are not limited to, acrylic acid, methacrylic acid, hydroxyethyl methacrylate, hydroxypropyl methacrylate, oligo(alkylene glycol)methyl ether (meth)acrylate (OAG(M)A), oligo(ethylene glycol) (meth)acrylate (OEG(M)A), acrylamide and methacrylamide, hydroxyethyl acrylate, N-methylacrylamide, H,N-dimethylacrylamide and N,N-dimethylaminoethyl methacrylate, N,N-dimethylaminopropyl methacrylamide, N-hydroxypropyl methacrylamide, 4-acryloylmorpholine, 2-acrylamido-2-methyl-1-propanesulfonic acid, phosphorylcholine methacrylate and N-vinyl pyrolidone.

Where the monomer used gives rise to a cationic homopolymer chain, as previously outlined, the so formed homopolymer chain may not inherently be in a charged cationic state. In other words, the homopolymer chain may need to be reacted with one or more other compounds to be converted into a charged cationic state. For example, the monomer selected to form a cationic homopolymer chain may comprise a tertiary amine functional group. Upon polymerising the monomer to form the cationic homopolymer chain, the tertiary amine functional group can be subsequently quaternarised into a positively charged state.

As a guide only, examples of cationic ethylenically unsaturated monomers include, but are not limited to, N,N-dimethyaminoethyl methacrylate, N,N-diethyaminoethyl methacrylate, N,N-dimethylaminoethyl acrylate, N,N-diethylaminoethyl acrylate, 2-aminoethyl methacrylate hydrochloride, N-[3-(N,N-dimethylamino)propyl]methacrylamide, N-(3-aminopropyl)methacrylamide hydrochloride, N-[3-(N,N-dimethylamino)propyl] acrylamide, N-[2-(N,N-dimethylamino)ethyl]methacrylamide, 2-N-morpholinoethyl acrylate, 2-N-morpholinoethyl methacrylate, 2-(N,N-dimethylamino)ethyl acrylate, 2-(N,N-dimethylamino)ethyl methacrylate, 2-(N,N-diethylamino)ethyl methacrylate, 2-acryloxyyethyltrimethylammonium chloride, mthacrylamidopropyltrimethylammonium chloride, 2-(tert-butylamino)ethyl methacrylate, allyldimethylammonium chloride, 2-(dethylamino)ethylstyrene, 2-vinylpyridine, and 4-vinylpyridine.

As a guide only, examples of hydrophobic ethylenically unsaturated monomers include, but are not limited to, styrene, alpha-methyl styrene, butyl acrylate, butyl methacrylate, amyl methacrylate, hexyl methacrylate, lauryl methacrylate, stearyl methacrylate, ethyl hexyl methacrylate, crotyl methacrylate, cinnamyl methacrylate, oleyl methacrylate, ricinoleyl methacrylate, cholesteryl methacrylates, cholesteryl acrylate, vinyl butyrate, vinyl tert-butyrate, vinyl stearate and vinyl laurate.

In the case of the hydrophilic ethylenically unsaturated monomer OAG(M)A, the alkylene moiety will generally be a C₂-C₆, for example a C₂ or C₃, alkylene moiety. Those skilled in the art will appreciate that the "oligo" nomenclature associated with the "(alkylene glycol)" refers to the presence of a plurality of alkylene glycol units. Generally, the oligo component of the OAG(M)A will comprise about 2 to about 200, for example from about 2 to about 100, or from about 2 to about 50 or from about 2 to about 20 alkylene glycol repeat units.

The hydrophilic homopolymer chain may therefore be described as comprising the polymerised residues of hydrophilic ethylenically unsaturated monomer.

The cationic homopolymer chain may therefore be described as comprising the polymerised residues of cationic ethylenically unsaturated monomer.

The hydrophobic homopolymer chain may therefore be described as comprising the polymerised residues of hydrophobic ethylenically unsaturated monomer.

Where a free radical polymerisation technique is to be used in polymerising ethylenically unsaturated monomer so as to form at least part of the branched polymer, the polymerisation will usually require initiation from a source of free radicals.

A source of initiating radicals can be provided by any suitable means of generating free radicals, such as the thermally induced homolytic scission of suitable compound(s) (thermal initiators such as peroxides, peroxyesters, or azo compounds), the spontaneous generation from monomers (e.g. styrene), redox initiating systems, photochemical initiating systems or high energy radiation such as electron beam, X- or gamma-radiation. Examples of such initiators may be found in, for example, WO 2010/083569 and Moad and Solomon "The Chemistry of Free Radical Polymerisation", Pergamon, London, 1995, pp 53-95.

The branched polymer may be constructed using techniques know in the art. For example, the homopolymer arms of the polymer may be first formed using an appropriate polymerisation reaction and then subsequently coupled to a suitable support moiety. This technique is known as a "coupling onto" approach. Such a coupling onto approach may involve coupling preformed homopolymer chains to a preformed support moiety.

Alternatively, preformed homopolymer chains may be coupled during the simultaneous formation of the support moiety. For example, preformed homopolymer chains having living polymerisation agent (or moiety) covalently bound thereto may be used to control the polymerisation of multi-ethylenically unsaturated monomer so as to form a crosslinked polymer support moiety to which is covalently attached the homopolymer chains.

To further illustrate how a branched polymer in accordance with the invention may be prepared, reference is made to Figure 2 in which 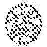 represents the support moiety in the form of a crosslinked polymer structure, - represents a general covalent bond, 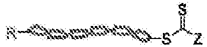 represents a cationic homopolymer chain having a RAFT agent covalently bound thereto, 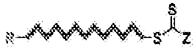 represents a hydrophilic homopolymer chain having a RAFT agent covalently bound thereto, and 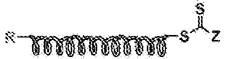 represents a hydrophobic homopolymer chain having a RAFT agent covalently bound thereto, where R and Z are as herein defined in the context of suitable RAFT agents.

With reference to Figure 2, preformed macro-RAFT agents in the form of a cationic homopolymer chain, a hydrophilic homopolymer chain, and a hydrophobic homopolymer chain are used to control the polymerisation of multi-ethylenically unsaturated monomer (DSDMA) so as to form a crosslinked polymer support moiety 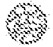 to which is covalently attached the homopolymer chains.

The present invention therefore also provides a method of preparing branched polymer comprising a support moiety and at least three homopolymer chains each covalently coupled to and extending from the moiety, wherein the at least three homopolymer chains include a cationic homopolymer chain, a hydrophilic homopolymer chain, and a hydrophobic homopolymer chain, the method comprising:
(i) providing a cationic homopolymer chain, a hydrophilic homopolymer chain, and a hydrophobic homopolymer chain, each homopolymer chain having a living polymerisation moiety covalently coupled thereto; and
(ii) polymerising one or more multi-ethylenically unsaturated monomers under the control of the living polymerisation moieties so as to form a crosslinked polymer support moiety to which is covalently attached each of the cationic, hydrophilic, and hydrophobic homopolymer chains.

In one embodiment of the method, each homopolymer chain provided in step (i) has a RAFT moiety covalently coupled thereto;
As used herein, a "living polymerisation moiety(ies)" is intended to mean a moiety that can participate in and control the living polymerisation of one or more ethylenically unsaturated monomers so as to form a living polymer chain.

Living polymerisation moieties suitable for use in accordance with the invention include, but are not limited to those which promote living polymerisation techniques selected from ionic polymerisation and controlled radial polymerisation (CRP). Examples of CRP include, but arc not limited to, iniferter polymerisation, stable free radical mediated polymerisation (SFRP), atom transfer radical polymerisation (ATRP), and reversible addition fragmentation chain transfer (RAFT) polymerisation as herein described.

Examples of multi-ethylenically unsaturated monomers (or multifunctional monomers) that may be used in accordance with the invention include disulfide dimethacrylates, disulphide dimethacrylates, ethylene glycol di(metli)aerylate, ethylene glycol diacrylate, triethylene glycol di(meth)acrylate, tetraethylene glycol di(meth)acrylate, poly(ethylene glycol) dimethacrylate, 1,3-butylene glycol di(meth)acrylate, trimethylolpropane tri(meth)acrylate, 1,4-butanediol di(meth)acrylate, 1,4-butanediol diacrylate, neopentyl glycol di(meth)acrylate, 1,6-hexanediol di(meth)acrylate, pentaerythritol di(meth)acrylate, pentaerythritol tri(meth)acrylate, pentaerythritol tetra(meth)acrylate, glycerol di(meth)acrylate, glycerol allyloxy di(meth)acrylate, 1,1,1-tris(hydroxymethyl)ethane di(meth)acrylate, 1,1,1-tris(hydroxymethyl)ethane tri(meth)acrylate, 1,1,1-tris(hydroxymethyl)propane di(meth)acrylate, 1,1,1-tris(hydroxymethyl)propane tri(meth)acrylate, triallyl cyanurate, triallyl isocyanurate, triallyl trimellitate, diallyl phthalate, diallyl terephthalte, divinyl benzene, divinyl adipate, 4,4'-divinylbiphenyl, methylol (meth)acrylamide, triallylamine, oleyl maleate, glyceryl propoxy triacrylate, allyl methacrylate, methacrylic anhydride and methylenebis (meth) acrylamide, bis(2-methacryloyl)oxyethyl disulphide, N,N'-bis(acryloyl)cystamine, and N,N'-methylenebisacrylarnide.

The branched polymer may also be formed by polymerising monomer directly from a suitable support moiety. This technique is known as a "core first" approach.

It may also be possible to use a combination of coupling onto and core first approaches. For example, monomer may be polymerised directly from a suitable support moiety to form the cationic homopolymer chain (core first). Preformed hydrophilic and hydrophobic homopolymer chains may then be coupled to the support moiety (coupling onto).

The present invention also provides a complex comprising the branched polymer and a nucleic acid molecule. The term "complex" as used herein refers to the association by ionic bonding of the branched polymer and the nucleic acid molecule. The ionic bonding is derived through electrostatic attraction between oppositely charged ions associated with the cationic homopolymer chain(s) of the branched polymer and the nucleic acid molecule. It will be appreciated that the cationic homopolymer chain will provide for positive charge, and accordingly the nucleic acid molecule will provide for negative charge so as to promote the required electrostatic attraction and formation of the complex.

The net negative charge on the nucleic acid molecule will generally be derived from the negatively charged nucleic acids *per se* (*e.g.* from the phosphate groups). Any modifications) made to the nucleic acid molecule should retain a net negative charge to the extent that it allows formation of a complex through ionic bonding with the branched polymer.

Without wishing to be limited by theory, the branched polymer and nucleic acid molecule are believed to form nanoparticles through ionic interactions between the negatively charged backbone of the nucleic acid molecule and the cationic homopolymer chain of the branched polymer. Depending on the number of cationic charges in a given branched polymer, one or more nucleic acid molecules may associate with the polymer to form complexes, and the number of the complexed nucleic acid molecules may increase with the increasing number of a arms/branches in the polymer. Accordingly, a branched polymer may have advantages in that more nucleic acid molecules can be complexed per branched polymer molecule than their linear counterparts.

The complex comprising the branched polymer and nucleic acid molecule may be prepared using known techniques for preparing cationic polymer/nucleic acid molecule complexes. For example, a required amount of polymer suspended in water may be introduced to a container comprising reduced serum media such as Opti-MEM®. The required amount of nucleic acid molecule may then be introduced to this solution and the resulting mixture vortexed for an appropriate amount of time so as to form the complex.

The nucleic acid molecule may be obtained commercially or prepared or isolated using techniques well known in the art.

There is no particular limitation concerning the ratio of nucleic acid molecule to branched polymer that may be used to form the complex. Those skilled in the art will appreciate that charge density (as indicated by zeta potential) of the branched polymer and nucleic acid, molecule, together with the ratio of branched polymer and nucleic acid molecule, will effect the overall charge/neutral state of the resulting complex.

In one embodiment, the complex has a positive Zeta potential. In a further embodiment, the complex has a positive Zeta potential ranging from greater than 0 mV to about 100mV. or from greater than 0 mV to about 50mV, for example from about 10mV to about 40mV, or from about 15 mV to about 30 mV, or from about 20 mV to about 25 mV.

The Zeta potential of a complex in accordance with the present invention is that as measured by Malvern Zetasizer. The Zeta potential is calculated from the measurement of the mobility of particles (electrophoertic mobility) in an electrical field and the particle size distribution in the sample.

The term "nucleic acid molecule" used 1-iereiii refers to nucleic acid molecules including DNA (gDNA, cDNA), oligonucleotides (double or single stranded), RNA (sense RNAs, antisense RNAs, mRNAs, tRNAs, rRNAs, small interfering RNAs (siRNAs), doublestranded RNAs (dsRNA), short hairpin RNAs (shRNAs), pi wi-interacting RNAs (PiRNA), micro RNAs (miRNAs), small nucleolar RNAs (SnoRNAs), small nuclear (SnRNAs) ribozymes, aptamers, DNAzymes, ribonuclease-type complexes and other such molecules as herein described. For the avoidance of doubt, the term "nucleic acid molecule" includes non-naturally occurring modified forms, as well as naturally occurring forms.

In some embodiments, the nucleic acid molecule comprises from about 8 to about 80 nucleobases (*i*.*e*. from about 8 to about 80 consecutively linked nucleic acids). One of ordinary skill in the art will appreciate that the present invention embodies nucleic acid molecules of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 nucleobases in length.

The term "nucleic acid molecule" also includes other families of compounds such as oligonucleotide analogs, chimeric, hybrid and mimetic forms.

Chimeric oligomeric compounds may also be formed as composite structures of two or more nucleic acid molecules, including, but not limited to, oligonucleotides, oligonucleotide analogs, oligonucleotides and oligonucleotide mimetics. Routinely used chimeric compounds include but are not limited to hybrids, hemimers, gapmers, extended gapmers, inverted gapmers and blockmers, wherein the various point modifications and or regions are selected from native or modified DNA and RNA type units and/or mimetic type subunits such as, for example, locked nucleic acids (LNA), peptide nucleic acids (PNA), morpholinos, and others. The preparation of such hybrid structures is described for example in US Pat. Nos. 5,013,830; 5,149,797; 5,220,007; 5,256,775; 5,366,878; 5,403,711; 5,491,133; 5,565,350; 5,623,065; 5,652,355; 5,652,356; and 5,700,922.

RNA and DNA aptamers are also contemplated. Aptamers are nucleic acid molecules having specific binding affinity to non-nucleic acid or nucleic acid molecules through interactions other than classic Watson-Crick base pairing. Aptamers are described, for example, in United States Patent Nos. 5,475,096; 5,270,163; 5,589,332; 5,589,332; and 5,741,679. An increasing number of DNA and RNA aptamers that recognize their non-nucleic acid targets have been developed and have been characterized (see, for example, Gold et al., Annu. Rev. Biochem., 64: 763-797,1995; Bacher et al., Drug Discovery Today, 3(6): 265-273, 1998).

Further modifications can be made to the nucleic acid molecules and may include conjugate groups attached to one of the termini, selected nucleobase positions, sugar positions or to one of the internucleoside linkages.

The present invention also provides a method of delivering a nucleic acid molecule to a cell, the method comprising:
(a) providing a complex comprising a branched polymer and a nucleic acid molecule, the branched polymer comprising a support moiety and at least three homopolymer chains covalently coupled to and extending from the moiety, wherein the at least three homopolymer chains include a cationic homopolymer chain, a hydrophilic homopolymer chain, and a hydrophobic homopolymer chain: and
(b) delivering the complex to the cell.

This method may be performed *in vivo*, *ex vivo* or *in vitro*.

The present invention further provides a method of gene therapy comprising the administration to a subject in need thereof a therapeutically effective amount of the nucleic acid molecule complex according to the present invention, as herein described.

The relevance of DNA repair and mediated recombination as gene therapy is apparent when studied, for example, in the context of genetic diseases such as cystic fibrosis, hemophilia and globinopathies such as sickle cell anemia and beta-thalassemia. For example, if the target gene contains a mutation that is the cause of a genetic disorder, then delivering a nucleic acid molecule into the cell(s) of a subject can be useful for facilitating mutagenic repair to restore the DNA sequence of the abnormal target gene to normal. Alternatively, the nucleic acid molecule introduced to the cell(s) of a subject may lead to the expression of a gene that is otherwise suppressed or silent in the disease state. Such nucleic acid molecules may themselves encode the silent or suppressed gene, or they may activate transcription and/or translation of an otherwise suppressed or silent target gene.

It would be understood by those skilled in the art that the disease or condition to be treated using the method of the present invention may be any disease or condition capable of treatment by gene therapy and the choice of the genetic material (*i.e*., nucleic acid molecule) to he used will clearly depend upon the particular disease or condition. Diseases or conditions that may be treated include, but are not limited to, cancers (*e*.*g*. myeloid disorders), thalassemia, cystic fibrosis, deafness, vision disorders (*e.g.* Leber's congenital amaurosis), diabetes, Huntingdon's disease, X-linked severe combined immunodeficiency disease and heart disease. Alternatively, the gene therapy may be used to introduce non-endogenous genes, for example, genes for bioluminescence, or to introduce genes which will knock out endogenous genes (*e.g.* RNA interference).

It would also be understood by those skilled in the art that the nature of the nucleic acid molecule will invariably depend on the disease or condition to be treated or prevented. For example, a disease or condition that is attributed, at least in part, to an accumulation of fibrotic extracellular matrix material (*e*.*g*., type II collagen), can be treated or prevented by delivering the nucleic acid molecule complex of the present invention to the subject (in a targeted or non-targeted approach), wherein the nucleic acid molecule (*e*.*g*., siRNA) is capable of silencing the gene that encodes the extracellular matrix material. In some embodiments, the disease or condition is an infectious disease, an inflammatory disease, or a cancer.

Where delivery of the nucleic acid molecule complex to a cell in accordance with the present invention is performed *in vivo*, the nucleic acid molecule complex can be introduced to the cell by any route of administration that is appropriate under the circumstances. For instance, where systemic delivery is intended, the complex may be administered intravenously, subcutaneously, intramuscularly, orally, *etc.* Alternatively, the complex may be targeted to a particular cell or cell type by means known to those skilled in the art. Targeting may be desirable for a variety of reasons such as, for example, to target cancer cells if the nucleic acid molecule is unacceptably toxic to non-cancerous cells or if it would otherwise require too high a dosage.

Targeted delivery may be performed *in vivo*, *ex vivo* or *in vitro* and achieved by means know to those skilled in the art. For example, this might be achieved via receptor-mediated targeting or by administering the nucleic acid complex directly to the tissue comprising the target cell(s).

Receptor-mediated targeting may be achieved by coupling or conjugating the branched polymer and/or nucleic acid molecule with a suitable targeting ligand as herein described.

For example, receptor-mediated targeting may be achieved by conjugating the nucleic acid molecule to a protein ligand, *e*.*g*., *via* polylysine.

Targeting ligands are typically chosen on the basis of the presence of the corresponding ligand receptors on the surface of the target cell/tissue type.

A ligand-nucleic acid molecule conjugate can be complexed with a branched polymer in accordance with the present invention and administered systemically if desired (*e*.*g*., intravenously), where they will be directed to the target cell/tissue where receptor binding occurs.

In one embodiment, the branched polymer and/or nucleic acid molecule is conjugated with a targeting ligand to promote receptor mediated targeting of the cell.

In another embodiment, the nucleic acid molecule is conjugated with a protein ligand to promote receptor mediated targeting of the cell.

The terms "coupled", "coupling" and "conjugated", "conjugating", are used interchangeably herein and are intended to mean that at least two entities are joined. typically by way of a covalent bond, so as to form a single entity.

In another embodiment, the method of delivering a nucleic acid molecule to a cell in accordance with the present invention is performed *ex vivo*. For example, cells are isolated from the subject and introduced *ex vivo* with the nucleic acid molecule complex of the present invention to produce cells comprising the exogenous nucleic acid molecule. The cells may be isolated from the subject to be treated or from a syngeneic host. The cells are then reintroduced back into the subject (or into a syngeneic recipient) for the purpose of treatment or prophyaxis. In some embodiments, the cells can be hematopoietic progenitor or stem cells.

In one embodiment, the nucleic acid molecule is delivered to a cell for the purpose of silencing (or suppressing) gene expression. In some embodiments. gene expression is silenced by reducing translational efficiency or reducing message stability or a combination of these effects. In some embodiments, splicing of the unprocessed RNA is the target goal leading to the production of non-functional or less active protein.

In some embodiments, gene expression is silenced by delivering to a cell a DNA molecule, including but not limited to, gDNA, cDNA and DNA oligonucleotides (double or single stranded).

In some embodiments, gene expression is silenced by RNA interference (RNAi). Without limiting the present invention to a particular theory or mode of action. "RNA interference" typically describes a mechanism of silencing gene expression that is based on degrading or otherwise preventing the translation of mRNA, for example, in a sequence specific manner. It would be understood by those skilled in the art that the exogenous interfering RNA molecules may lead to either mRNA degradation or mRNA translation repression. In some embodiments, RNA interference is achieved by altering the reading frame to introduce one or more premature stop codons that lead to non-sense mediated decay.

RNAi includes the process of gene silencing involving double stranded (sense and antisense) RNA that leads to sequence specific reduction in gene expression *via* target mRNA degradation. RNAi is typically mediated by short double stranded siRNAs or single stranded microRNAs (miRNA).

Other oligonucleotides having RNA-like properties have also been described and many more different types of RNAi may be developed. For example, antisense oligonucleotides have been used to alter exon usage and to modulate pre-RNA splicing (see, for example, Madocsai et al., Molecular Therapy, 12: 1013-1022, 2005 and Aartsma-Rus et al., BMC Med Genet., 8: 43, 2007). Antisense and iRNA compounds may be double stranded or single stranded oligonucleotides which are RNA or RNA-like or DNA or DNA-like molecules that hybridize specifically to DNA or RNA of the target gene of interest:
Examples of RNA molecules suitable for use in the context of the present invention include, but are not limited to: *long double stranded RNA* (*dsRNA*); *hairpin double stranded RNA* (*hairpin dsRNA*); *short interfering RNA (siRNA)*, *short hairpin RNA (shRNA micro RNA*/*small temporal RNA (miRNA*/*siRNA)*; miRNAs which mediate spatial development (sdRNAs), the stress response (srRNAs) or cell cycle (ccRNAs); and RNA oligonucleotides designed to hybridise and prevent the functioning of endogenously expressed miRNA or stRNA or exogenously introduced siRNA.

In other embodiments, the nucleic acid molecule suppresses translation initiation, splicing at a splice donor site or splice acceptor site. In other embodiments, modification of splicing alters the reading frame and initiates nonsense mediated degradation of the transcript.

In another example pertaining to the design of a nucleic acid molecule suitable for use in accordance with the present invention, it is within the skill of the person of skill in the art to determine the particular structure and length of the molecule, for example whether it takes the form of dsRNA, hairpin dsRNA, siRNA, shRNA, miRNA, pre-miRNA, pri-miRNA or any other suitable form as herein described.

The term "gene" is used in its broadest sense and includes cDNA corresponding to the exons of a gene. Reference herein to a "gene" is also taken to include; a classical genomic gene consisting of transcriptional and/or translational regulatory sequences and/or a coding region and/or non-translated sequences (*i*.*e*. introns, 5'- and 3'- untranslated sequences); or an mRNAor cDNA molecule corresponding to the coding regions (*i.e.* exons), pre-mRNA and 5'- and 3'- untranslated sequences of the gene.

Reference to "expression" is a broad reference to gene expression and includes any stage in the process of producing protein or RNA from a gene or nucleic acid molecule, from pre-transcription, through transcription and translation to post-translation.

A "cell", as used herein, includes a eukaryotic cell (*e.g*., animal cell, plant cell and a cell of fungi or protists) and a prokaryotic cell (*e.g*., a bacterium). In one embodiment, the cell is a human cell.

The term "subject", as used herein, means either an animal or human subject. By "animal" is meant primates, livestock animals (including cows, horses, sheep, pigs and goats), companion animals (including dogs, eats, rabbits and guinea pigs), captive wild animals (including those commonly found in a zoo environment), and aquatic animals (including freshwater and saltwater animals such as fish and crustaceans, Laboratory animals such as rabbits, mice, rats, guinea pigs and hamsters are also contemplated as they may provide a convenient test system. In some embodiments. the subject is a human subject.

By "administration" of the complex or composition to a subject is meant that the agent or composition is presented such that it can be or is transferred to the subject. There is no particular limitation on the mode of administration, but this will generally be by way of oral, parenteral (including subcutaneous, intradermal, intramuscular, intravenous, intrathecal, and intraspinal), inhalation (including nebulisation), rectal and vaginal modes.

Without being bound or limited by theory, the complex of the present invention has been found to protect the nucleic acid molecule from degradation by enzymes such as RNAse and/or DNAse.

The present invention therefore also provides a method of protecting a nucleic acid molecule form enzymatic degradation, the method comprising complexing the nucleic acid molecule with a branched polymer comprising a support moiety and at least three homopolymer chains covalently coupled to and extending from the moiety, wherein the at least three homopolymer chains include a cationic homopolymer chain, a hydrophilic homopolymer chain, and a hydrophobic homopolymer chain.

There is also provided use of a complex for delivering a nucleic acid molecule to a cell, the complex comprising a branched polymer and the nucleic acid molecule, the branched polymer comprising a support moiety and at least three homopolymer chains covalently coupled to and extending from the moiety, wherein the at least three homopolymer chains include a cationic homopolymer chain, a hydrophilic homopolymer chain, and a hydrophobic homopolymer chain.

The present invention further provides use of a complex for silencing gene expression, the complex comprising a branched polymer and a nucleic acid molecule, the branched polymer comprising a support moiety and at least three homopolymer chains covalently coupled to and extending from the moiety, wherein the at least three homopolymer chains include a cationic homopolymer chain, a hydrophilic homopolymer chain, and a hydrophobic homopolymer chain.

In one embodiment, the nucleic acid molecule is selected from DNA and RNA. In a further embodiment, the DNA and RNA are selected from gDNA, cDNA, double or single stranded DNA oligonucleotides, sense RNAs, antisense RNAs, mRNAs, tRNAs, rRNAs, small/short interfering RNAs (siRNAs), double-stranded RNAs (dsRNA), short hairpin RNAs (shRNAs), piwi-interacting RNAs (PiRNA), micro RNA/small temporal RNA (miRNA/stRNA), small nucleolar RNAs (SnoRNAs), small nuclear (SnRNAs) ribozymes, aptamers, DNAzymes, ribonuclease-type complexes, hairpin double stranded RNA (hairpin dsRNA), miRNAs which mediate spatial development (sdRNAs), stress response RNA (srRNAs), cell cycle RNA (ccRNAs) and double or single stranded RNA oligonucleotides.

The present invention still further provides use of a branched polymer in protecting a nucleic acid molecule from enzymatic degradation, the branched polymer comprising a support moiety and at least three homopolymer chains covalently coupled to and extending from the moiety, wherein the at least three homopolymer chains include a cationic homopolymer chain, a hydrophilic homopolymer chain, and a hydrophobic homopolymer chain.

The present invention is also directed to compositions, such as pharmaceutical compositions, comprising the nucleic acid molecule complex of the present invention. In some embodiments, the composition will comprise the nucleic acid molecule complex of the present invention and one or more pharmaceutically acceptable carriers, diluents and/or excipients.

In the compositions of the present invention, the nucleic acid molecule complex is typically formulated for administration in an effective amount. The terms "effective amount" and "therapeutically effective amount" of the nucleic acid complex as used herein typically mean a sufficient amount of the complex to provide in the course the desired therapeutic or prophylactic effect in at least a statistically significant number of subjects.

In some embodiments, an effective amount for a human subject lies in the range of about 0.1ng/kg body weight/dose to 1g/kg body weight/dose. In some embodiments, the range is about 1µg to 1g, about 1mg to 1g, 1mg to 500mg, 1mg to 250mg, 1mg to 50mg, or 1µg to 1mg/kg body weight/dose. Dosage regimes are adjusted to suit the exigencies of the situation and may be adjusted to produce the optimum therapeutic or prophylactic dose.

By "pharmaceutically acceptable" carrier, excipient or diluent is meant a pharmaceutical vehicle comprised of a material that is not biologically or otherwise undesirable; that is, the material may be administered to a subject along with the complex of the present invention without causing any or a substantial adverse reaction.

Aspects of the present invention include methods for treating a subject for an infectious disease, an inflammatory disease, or a cancer, the method comprising administering to the subject a complex according to the invention, or a pharmaceutical composition according to the invention, to the subject.

The branched polymers according to the invention may also find use as delivery vehicles for bioactives in the agricultural sector, cosmetic sector, as viscosity modifiers, surfactants, dispersants, or as additives in, for example, formulations for paints and cosmetics.

As used herein, the term "alkyl", used either alone or in compound words denotes straight chain, branched or cyclic alkyl, preferably C₁₋₂₀ alkyl, e.g. C₁₋₁₀ or C₁₋₆. Examples of straight chain and branched alkyl include methyl, ethyl, *n*-propyl, isopropyl, *n*-butyl, *sec-*butyl, *i*-butyl, *n*-pentyl, 1,2-dimethylpropyl, 1,1-dimethyl-propyl, and hexyl. Examples of cyclic alkyl include mono- or polycyclic alkyl groups such as cyclopropyl, cyclobutyl, cyclopentyl, cyclohexyl, cycloheptyl, cyclooctyl, cyclononyl, cyclodecyl and the like. Where an alkyl group is referred to generally as "propyl", butyl" etc, it will be understood that this can refer to any of straight, branched and cyclic isomers where appropriate. An alkyl group may be optionally substituted by one or more optional substituents as herein defined.

The term "alkenyl" as used herein denotes groups formed from straight chain, branched or cyclic hydrocarbon residues containing at least one carbon to carbon double bond including ethylenically mono-, di- or polyunsaturated alkyl or cycloalkyl groups as previously defined, preferably C₂₋₂₀ alkenyl (e.g. C₂₋₁₀ or C₂₋₆). Examples of alkenyl include vinyl, allyl. 1-methylvinyl, and butenyl. An alkenyl group may be optionally substituted by one or more optional substituents as herein defined.

As used herein the term "alkynyl" denotes groups formed from straight chain, branched or cyclic hydrocarbon residues containing at least one carbon-carbon triple bond including ethylenically mono-, di- or polyunsaturated alkyl or cycloalkyl groups as previously defined. Unless the number of carbon atoms is specified the term preferably refers to C₂₋₂₀ alkynyl (e.g. C₂₋₁₀ or C₂₋₆). Examples include ethynyl, 1-propynyl, 2-propynyl, and butynyl isomers, and pentynyl isomers. An alkynyl group may be optionally substituted by one or more optional substituents as herein defined.

The term "halogen" ("halo") denotes fluorine, chlorine, bromine or iodine (fluoro, chloro, bromo or iodo).

The term "aryl" (or "carboaryl") denotes any of single, polynuclear, conjugated and fused residues of aromatic hydrocarbon ring systems(e.g. C₆₋₂₄ or C₆₋₁₈). Examples of aryl include phenyl, biphenyl, terphenyl, quaterphenyl and naphthyl. An aryl group may or may not be optionally substituted by one or more optional substituents as herein defined. The term "arylene" is intended to denote the divalent form of aryl.

The term "carbocyclyl" includes any of non-aromatic monocyclic, polycyclic, fused or conjugated hydrocarbon residues, preferably C₃₋₂₀ (e.g. C₃₋₁₀ or C₃₋₈). The rings may be saturated, e.g. cycloalkyl, or may possess one or more double bonds (cycloalkenyl) and/or one or more triple bonds (cycloalkynyl). A carbocyclyl group may be optionally substituted by one or more optional substituents as herein defined. The term "carbocyclylene" is intended to denote the divalent form of carbocyclyl.

The term "heteroatom" or "hetero" as used herein in its broadest sense refers to any atom other than a carbon atom which may be a member of a cyclic organic group. Particular examples of heteroatoms include nitrogen, oxygen, sulfur, phosphorous, boron, silicon, selenium and tellurium, more particularly nitrogen, oxygen and sulfur.

The term "heterocyclyl" when used alone or in compound words includes any of monocyclic, polycyclic, fused or conjugated hydrocarbon residues, preferably C₃₋₂₀ (e.g. C₃₋₁₀ or C₃₋₈) wherein one or more carbon atoms are replaced by a heteroatom so as to provide a non-aromatic residue. Suitable heteroatoms include O, N, S, P and Se, particularly O, N and S. Where two or more carbon atoms are replaced, this may be by two or more of the same heteroatom or by different heteroatoms. The heterocyclyl group may be saturated or partially unsaturated, i.e. possess one or more double bonds. A heterocyclyl group may be optionally substituted by one or more optional substituents as herein defined. The term "heterocyclylene" is intended to denote the divalent form of heterocyclyl.

The term "heteroaryl" includes any of monocyclic, polycyclic, fused or conjugated hydrocarbon residues, wherein one or more carbon atoms are replaced by a heteroatom so as to provide an aromatic residue. Preferred heteroaryl have 3-20 ring atoms, e.g. 3-10. Particularly preferred heteroaryl are 5-6 and 9-10 membered bicyclic ring systems. Suitable heteroatoms include, O, N, S, P and Se, particularly O, N and S. Where two or more carbon atoms are replaced, this may be by two or more of the same heteroatom or by different heteroatoms. A heteroaryl group may be optionally substituted by one or more optional substituents as herein defined. The term "heteroarylene" is intended to denote the divalent form of heteroaryl.

The term "acyl" either alone or in compound words denotes a group containing the moiety C=O (and not being a carboxylic acid, ester or amide) Preferred acyl includes C(O)-R^{e}, wherein R^{e} is hydrogen or an alkyl, alkenyl, alkynyl, aryl, heteroaryl, carbocyclyl, or heterocyclyl residue.

The term "sulfoxide", either alone or in a compound word, refers to a group -S(O)R^{f} wherein R^{f} is selected from hydrogen, alkyl, alkenyl, alkynyl, aryl, heteroaryl, heterocyclyl, carbocyclyl, and aralkyl. Examples of preferred R^{f} include C₁₋₂₀alkyl, phenyl and benzyl.

The term "sulfonyl", either alone or in a compound word, refers to a group S(O)₂-R^{f}, wherein R^{f} is selected from hydrogen, alkyl, alkenyl, alkynyl, aryl, heteroaryl, heterocyclyl, carbocyclyl and aralkyl.

The term "sulfonamide", either alone or in a compound word, refers to a group S(O)NR^{f}R^{f} wherein each R^{f} is independently selected from hydrogen, alkyl, alkenyl, alkynyl, aryl, heteroaryl, heterocyclyl, carbocyclyl, and aralkyl.

The term, "amino" is used here in its broadest sense as understood in the art and includes groups of the formula NR^{a}R^{b} wherein R^{a} and R^{b} may be any independently selected from hydrogen, alkyl, alkenyl, alkynyl, aryl, carbocyclyl, heteroaryl, heterocyclyl, arylalkyl, and acyl. R^{a} and R^{b}. together with the nitrogen to which they are attached, may also form a monocyclic, or polycyclic ring system e.g. a 3-10 membered ring, particularly, 5-6 and 9-10 membered systems.

The term "amido" is used here in its broadest sense as understood in the art and includes groups having the formula C(O)NR^{a}R^{b}, wherein R^{a} and R^{b} are as defined as above.

The term "carboxy ester" is used here in its broadest sense as understood in the art and includes groups having the formula CO₂R^{g}, wherein R^{g} may be selected from groups including alkyl, alkenyl, alkynyl, aryl, carbocyclyl, heteroaryl, heterocyclyl, aralkyl, and acyl.

As used herein, the term "aryloxy" refers to an "aryl" group attached through an oxygen bridge. Examples of aryloxy substituents include phenoxy, biphenyloxy, naphthyloxy and the like.

As used herein, the term "acyloxy" refers to an "acyl" group wherein the "acyl" group is in turn attached through an oxygen atom.

As used herein, the term "alkyloxycarbonyl" refers to an "alkyloxy" group attached through a carbonyl group. Examples of "alkyloxycarbonyl" groups include butylformate, sec-butylformate, hexylformate, octylformate, decylformate, cyclopentylformate and the like.

As used herein, the term "arylalkyl" refers to groups formed from straight or branched chain alkanes substituted with an aromatic ring. Examples of arylalkyl include phenylmethyl (benzyl), phenylethyl and phenylpropyl.

As used herein, the term "alkylaryl" refers to groups formed from aryl groups substituted with a straight chain or branched alkane. Examples of alkylaryl include methylphenyl and isopropylphenyl.

In this specification "optionally substituted" is taken to mean that a group may or may not be substituted or fused (so as to form a condensed polycyclic group) with one, two, three or more of organic and inorganic groups, including those selected from: alkyl, alkenyl, alkynyl, carbocyclyl, aryl, heterocyclyl, heteroaryl, acyl, aralkyl, alkaryl, alkheterocyclyl, alkheteroaryl, alkarbocyclyl, halo, haloalkyl, haloalkenyl, haloalkynyl, haloaryl, halocarbocyclyl, haloheterocyclyl, haloheteroaryl, haloacyl, haloaryalkyl, hydroxy, hydroxyalkyl, hydroxyalkenyl, hydroxyalkynyl, hydroxycarbocyclyl, hydroxyaryl, hydroxyheterocyclyl, hydroxyheteroaryl, hydroxyacyl hydroxyaralkyl, alkoxyalkyl, alkoxyalkenyl, alkoxyalkynyl, alkoxycarbocyclyl, alkoxyaryl, alkoxyheterocyclyl, alkoxyheteroaryl, alkoxyacyl, alkoxyaralkyl, alkoxy, alkenyloxy, alkynyloxy, aryloxy, carbocyclyloxy, aralkyloxy, heteroaryloxy, heterocyclyloxy, acyloxy, haloalkoxy, haloalkenyloxy, haloalkynyloxy, haloaryloxy, halocarbocyclyloxy, haloaralkyloxy, haloheteroaryloxy, haloheterocyclyoxy, haloacyloxy, nitro, nitroalkyl, nitroalkenyl, nitroalkynyl, nitroaryl, nitroheterocyclyl, nitroheteroayl, nitrocarbocyclyl, nitroacyl, nitroaralkyl, amino (NH₂), alkylamino, dialkylamino, alkenylamino, alkynylamino, arylamino, diarylamino, aralkylamino, diaralkylamino, acylamino, diacylamino, heterocyclamino, heteroarylamino, carboxy, carboxyester, amido, alkylsulphonyloxy, arylsulphenyloxy, alkylsulphenyl, arylsulphenyl, thio, alkylthio, alkenylthio, alkynylthio, arylthio, aralkylthio, carbocyclylthio, heterocyclylthio, heteroarylthio, acylthio, sulfoxide, sulfonyl, sulfonamide, aminoalkyl, aminoalkenyl, aminoalkynyl, aminocarbocyclyl, aminoaryl, aminoheterocyclyl, aminoheteroaryl, aminoacyl, aminoaralkyl, thioalkyl, thioalkenyl, thioalkynyl, thiocarbocyclyl, thioaryl, thioheterocyclyl, thioheteroaryl, thioacyl, thioaralkyl, carboxyalkyl, carboxyalkenyl, carboxyalkynyl, carboxycarbocyclyl, carboxyaryl, carboxyheterocyclyl, carboxyheteroaryl, carboxyacyl, carboxyaralkyl, carboxyesteralkyl, carboxyesteralkenyl, carboxyesteralkynyl, carboxyestercarbocyclyl, carboxyesteraryl, carboxyesterheterocyclyl, carboxyesterheteroaryl, carboxyesteracyl, carboxyesteraralkyl, amidoalkyl, amidoalkenyl, amidoalkynyl, amidocarbocyclyl, amidoaryl, amidoheterocyclyl, amidoheteroaryl, amidoacyl, amidoaralkyl, formylalkyl, formylalkenyl, formylalkynyl, formylcarbocyclyl, formylaryl, formylheterocyclyl, formylheteroaryl, formylacyl, formylaralkyl, acylalkyl, acylalkenyl, acylalkynyl, acylcarbocyclyl, acylaryl, acylheterocyclyl, acylheteroaryl, acylacyl, acylaralkyl. sulfoxidealkyl, sulfoxidealkenyl, sulfoxidealkynyl, sulfoxidecarbocyclyl, sulfoxidearyl, sulfoxideheterocyclyl, sulfoxideheteroaryl, sulfoxideacyl, sulfoxidearalkyl, sulfonylalkyl, sulfonylalkenyl, sulfonylalkynyl, sulfonylearbocyclyl, sulfonylaryl, sulfonylheterocyclyl, sulfonylheteroaryl, sulfonylacyl, sulfonylaralkyl, sulfonamidoalkyl, sulfonamidoalkenyl, sulfonamidoalkynyl, sulfonamidocarbocyclyl, sulfonamidoaryl, sulfonamidoheterocyclyl, sulfonamidoheteroaryl, sulfonamidoacyl, sulfonamidoaralkyl, nitroalkyl, nitroalkenyl, nitroalkynyl, nitrocarbocyclyl, nitroaryl, nitroheterocyclyl, nitroheteroaryl, nitroacyl, nitroaralkyl, cyano, sulfate, phosphate, triarylmethyl, triarylamino, oxadiazole, and carbazole groups. Optional substitution may also be taken to refer to where a -CH₂- group in a chain or ring is replaced by a group selected from -O-, -S-, -NR^{a}-, -C(O)- (i.e. carbonyl), -C(O)O- (i.e. ester), and -C(O)NR^{a}- (i.e. amide), where R^{a} is as defined herein.

The invention will now be described with reference to the following non-limiting examples.

### EXAMPLES

### Example 1

### Materials

Oligo(ethylene glycol) methacrylate (OEGMA₈₋₉, *Mn* ∼ 475 g,mol⁻¹), N,N-dimethylaminoethyl methacrylate (DMAEMA), *n*-butyl methacrylate (*n*-BMA) monomers and ethylene glycol dimethylacrylate (EGDMA) crosslinker were purchased from Aldrich and purified by stirring in the presence of inhibitor-remover for hydroquinone or hydroquinone monomethyl ether (Aldrich) for 30 min prior to use, 4-Cyano-4-[(dodecylsulfanylthiocarbonyl)sulfanyl]pentanoic acid (DTTCP) RAFT agent,(G. Moad, Y. K. Chong, A. Postma, E. Rizzardo, S. H. Thang, Polymer 2005, 46, 8458.)^{[1]} disulfide dimethacrylate (DSDMA) crosslinker (J. Rosselgong, S. P, Armes, W. Barton, D. Price, Macromolecules 2009, 42, 5919)^{[2]} were prepared according to the published literatures. 1,1'-azobis(cyclohexanecarbonitrile) (ACCN or VAZO-88, DuPont) initiator, tributylphosphine (Bu₃P, Aldrich) reductant was used as received. N,N-dimethylformamide (DMF), dichloromethane (DCM), n-hexane, n-heptane, diisopropyl ether, methanol, and other chemical substances were commercial reagents and used without further purification.

### Characterization:

Proton nuclear magnetic resonance (¹H NMR) spectra were obtained with a Bruker Advance 400 MHz spectrometer (¹H 400 MHz). Gel permeation chromatography (GPC) measurements were performed on a Shimadzu system equipped with a CMB-20A controller system, a SIL-20A HT autosampler, a LC-20AT tandem pump system, a DGU-20A degasser unit, a CTO-20AC column oven, a RDI-10A refractive index detector and with 4 × Waters Styragel columns (HT2, HT3, HT4, HT5 each 300 mm ×7.8 mm providing an effective molar mass range of 100-4000000), and uses N,N-dimethylacetamide (DMAc) (with 2.1 g L⁻¹ of lithium chloride (LiCl) as eluent with a flow rate of 1 mL min⁻¹ at 80 °C. The molar mass of the samples was obtained from a calibration curve constructed with poly(methyl methacrylate) (PMMA) standards (Polymer Laboratories) of low polydispersity index value. A third-order polynomial was used to fit the log *M*ₚ versus time calibration curve, which was linear across the molar mass ranges. Dynamic light scattering (DLS) experiments were performed using a Malvern Instruments Zetasizer Nanoseries instrument equipped with a 4 mW HeNe laser operating at 633 nm, an avalanche photodiode detector with high quantum effciency, and an ALV/LSE-5003 multiple τ digital correlator electronics system. Aqueous light scattering studies were performed on aqueous 1 mg.mL-1 star polymer solutions with a background electrolyte of 10 mM NaCl.

### Synthesis of the Dansyl RAFT-agent (3):

The precursor dansyl ethylenediamine (or 2-dansylammoethylamine) (**1**) for making the title dansyl-RAFT agent was prepared in 84.8% yield after recrystallisation in dichloromethane: n-hexane (1:1) solvent mixture according to a published literature by Schrader et. al., Chem. Eur. J. 2007, 13, 7701-7707.

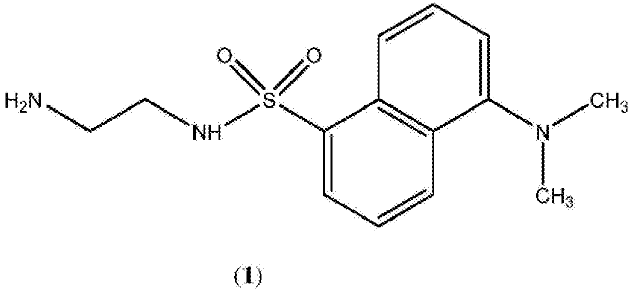

Dansyl-RAFT agent:

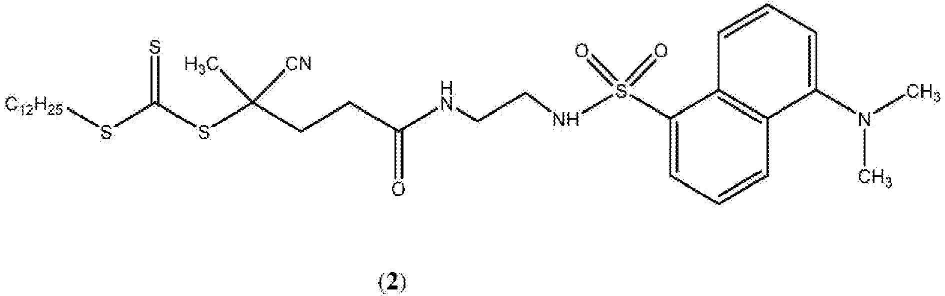

To a solution of dansyl ethylenediamine (293 mg, 1.0 mmol), 4-cyano-4-[(dodecylsulfanylthiocarbonyl)sulfanyl]pentanoic acid (2) (403 mg, 1.0 mmol) and catalyst *N,N*-dimethylaminopyridine (DMAP) in dichloromethane (5 mL) was added diisopropyl carbodiimide (DIC) (140 mg, 1.1 mmol), the reaction mixture was allowed to stir at room temperature for 4 h. The DIC-urea by-product was filtered, volatiles removed in vacuo and the crude reaction mixture (790mg) was purified by column chromatography (ethyl acetate: *n*-hexane 3:2 v/v as eluent) to give the title product dansyl-RAFT agent (**2**) as a yellow liquid (460 mg, 67.8 %). ¹H NMR (CDCl₃) δ(ppm) 0.88 (t, 3H, CH₃), 1.21 - 1. 40 (br.s, 18H, 9xCH₂), 1.70 (m, 2H, CH₂), 1.85 (s, 3H, CH₃), 2.30-2.42 (m, 4H, CH₂CH₂), 2.89 (s. 6H, N(CH₃)₂), 3.05 (m, 2H, C(=O)NH CH₂), 3.30 (m, 2H, S(O)₂NHCH₂), 3.33 (dd, 2H, CH₂S), 5.49 (t, 1H, NH), 5.99 (t, 1H, NH). 7.21 (d, 1H, Ar-H), 7.53 (dd, 1H, Ar-H), 7.60 (dd, 1H, Ar-H), 8.24 (dd, 1H, Ar-H), 8.55 (d, 1H, Ar-H).

### Synthesis of SN38 RAFT agent:

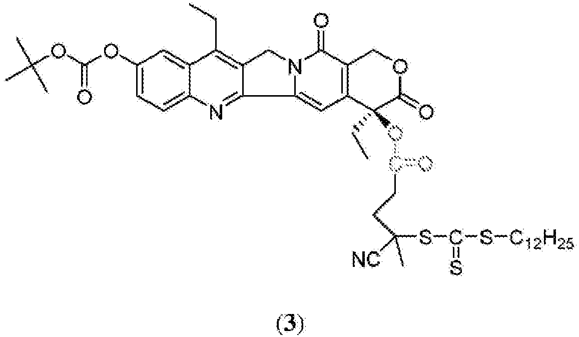

The SN38-RAFT agent was prepared in a two-step synthesis according to the published literature by Williams et al., Chem Med Chem, 2012, 7, 281-291. Firstly, the 10-Boc-SN38 (Boc-protected 10-OH group of SN38) was prepared from SN38 (SN38 is an anticancer drug) according to the procedure of Zhao et al., (Biocanjugate Chem. 2008, 19, 849-59) and then in the second step, the obtained 10-Boc-SN38 was allowed to react with 4-cyano-4-[(dodecylsulfanylthiocarbonyl)sulfanyl]pentanoic acid in the presence of DIC coupling reagent in dichloromethane solvent.

To a solution of 10-Boc-SN-38 (510 mg, 1.035 mmol), 4-cyano-4-[(dodecylsulfanylthiocarbonyl)sulfanyl]pentanoic acid (426 mg, 1.05 mmol) and catalyst *N,N*-dimethylaminopyridine in DCM (15 mL) was added a solution of diisopropyl carbodiimide (DIC) (195 mg, 1.56 mmol, 1.5 eq.) in DCM, the reaction mixture was allowed to stir at room temperature for 16 h. The DIC-urea by-product was filtered, volatiles removed in vacuo and the crude reaction mixture was purified by column chromatography (ethyl acetate: n-hexane 1:1 v/v as eluent) to give SN38-RAFT agent as a yellow solid (498 mg, 55 %). ¹H NMR (CDCl₃) δ(ppm) 0.84 (t. 3H, CH₃), 0.96 (t, 3H. CH₃), 1.21 - 1.36 (br.m, 18H, 9xCH₂), 1.35 (t, 3H, CH₃), 1.58 (s, 9H, 3xCH₃), 1.63 (m, 2H, CH₂), 1.81 (s, 3H, CH₃), 2.14 (m, 1H), 2.25 (m, 1H), 2.35 (m, 1H), 2.50 (m, 1H), 2.79 (m, 2H), 3.10 (dd, 2H, CH₂S), 3.21 (dt, 1H), 3.27 (t, 1H), 5.18 (s, 2H,), 5.37 (d, 1H,), 5.64 (d, 1H), 7.14 (d, 1H, Ar-H), 7.64 (dd, 1H, Ar-H), 7.84 (d, 1H, Ar-H), 8.21 (dd, 1H, Ar-H),

### Synthesis of mikto-arm star polymers by the arm first approach

**Polymerization kinetic protocol:** The polymerization rates of OEGMA₈₋₉, DMAEMA and BMA were ascertained by conducting separate polymerization experiments and the general procedure used is summarized below:
Stock solution I of VAZO-88 (25 mg) in DMF (2.5 g) was prepared in a flask. A mixture of DTTCP (106 mg), OEGMA₈₋₉ (5.0 g), stock solution I (643 mg) and DMF (4.5 g) was prepared in a second flask. Aliquots (1.0 g) of this stock solution were transferred to five ampoules which were degassed by three repeated freeze-evacuate-thaw cycles and sealed under vacuum. The ampoules were heated at 90 °C for the specified times and then subjected to GPC and ¹H NMR analysis. Figure 3 illustrates the growth of polymer molecular weights for the three monomers with respect to time and monomer conversion as well as the polymer dispersity. The GPC traces of the three polymers prepared are shown in Figure 3 (I) molecular weight results from GPC and polydispersity of the polymers, ***Ð***, are shown in Table 1.

**Table 1. Preparation of homopolymers of OEGMA₈₋₉, DMAEMA and n-BMA and their GPC molecular weight results**

| **Entry** | **Polymer composition** | **Time [h]** | **Conversion^{a)} [%]** | ***Mₙ* ^{b)} (Theo)** | ***Mₙ* ^{c)} (GPC)** | ***Ð*^{e)} (GPC)** |
|---|---|---|---|---|---|---|
| M-RAFT 1 | P(OEGMA₈₋₉) | 4 | 80.7 | 15,700 | 11,800) | 1.24 |
| M-RAFT 2 | P(DMAEMA) | 6 | 79.9 | 15,500 | 13,100 | 1.19 |
| M-RAFT 3 | P(*n*-BMA) | 8 | 73.8 | 14,000 | 11,500 | 1.11 |

| | | | | | | |
|---|---|---|---|---|---|---|
| ^{a)} Monomer conversions were calculated from ¹H NMR; ^{b)} *M*ₙ (Theo) were calculated from monomer conversion; ^{c)} *M*ₙ (GPC) and ***Ð*** (GPC) were obtained from DMAc GPC using PMMA standards. | | | | | | |

**Synthesis of mikto-arm polymers:** A typical procedure for the synthesis mikto-arm polymers using ampoules is as follows;
Stock solutions of ACCN (1.0 wt.%), P(OEGMA₈₋₉) (30,0 wt.%), P(DMAEMA) (30.1 wt.%), P(*n*-BMA) (27.3 wt.%), DSDMA (20.0 wt.%) in DMF were prepared in flasks. Specific amounts of each monomer ([Monomer]: [Polymer] =6:1) was added into polymer solution respectively. A mixture of ACCN solution (73 mg), P(OEGMA₈₋₉) solution (129 mg). P(DMAEMA) solution (127 mg), P(*n*-BMA) solution (120 mg), DSDMA solution (146 mg) and DMF (127 mg) was prepared in a flask. The stock solution was transferred to an ampoule which was degassed by three freeze-evacuate-thaw cycles and sealed under vacuum. The ampoule was heated at 90 °C for 20h.

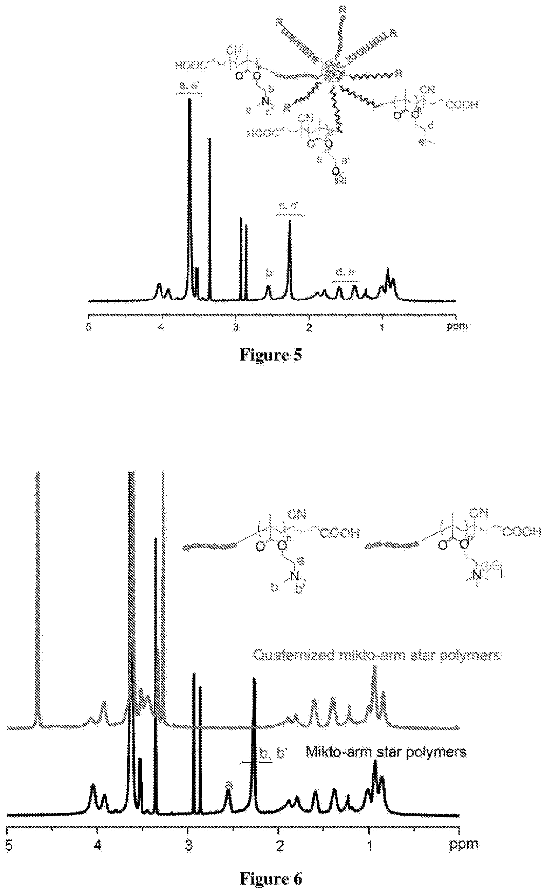

Seven star polymers were prepared according to this procedure and Table 2 summarizes the relative amounts of the each homopolymer macro RAFT agents and other reagents used as well as the mikto-arm polymer molecular weights based on GPC analysis. The GPC traces of selected mikto-arm polymers are shown in Figure 4 (III). Figure 4 (IV) shows the GPC traces of the mixed arms before polymerization, after polymerization (crude polymer), purified polymer, and the degraded polymer. Figure 5 illustrates the ¹H-NMR spectrum of the purified mikto-arm polymer.

### Quaternization of the mikto-arm star polymers:

To quaternize the tertiary amino group of P(DMAEMA) arm of the mikto-arm star polymers, a stock solution of the stars mention above was diluted with DMF, then an excess of Mel was added into the solution and stirred for 16 h at room temperature. Finally, the excess of MeI was removed on a rotary evaporator; the DMF was removed by dialysis of the star polymers against water for 4 days (molecular weight membrane cut off 25,000 Da). The star polymers containing quaternized P(DMAEMA) were obtained after freeze-drying. Figure 6 shows the ¹H-NMR spectrum of the quaternized polymer.

**Table 2. Summary of the composition, polymerization time, monomer and arm conversion and molecular weight data (DMAc using PMMA standards) for the mikto-arm star polymers prepared according to the procedure described in Example 1.**

| **Mikto-arm Polymer Code** | **Composition** | **Arm Mn** | **[DSDMA] /[M-CTA]** | **M-CTA ratio** | **Arm conversion^{a}** | **Mn (star)^{b)}** | **PDI** | **Mn (star cleaved)** |
|---|---|---|---|---|---|---|---|---|
| S3-16 | POEGMA-PQDMAEMA-PBMA star | 15k/15k/15k | 12 | 3/3/3 | 81.5 | 136,200 | 1.33 | 17,800 |
| S4-1 | Dansyl-POEGMA-Dansyl-PQDMAEMA-Dansyl-PBMA star | 15k/15k/15k | 12 | 3/3/3 | 89.1 | 87,400 | 1.19 | 20,800 |
| S4-3 | Dansyl-POEGMA-Dansyl-PQDMAEMA-Dansyl-PBMA star | 5k/5k/5k | 8 | 3/3/3 | 90.4 | 47.100 | 1.17 | 10,700 |
| S4-4 | SN38-POEGMA-SN38 - PQDMAEMA-SN38 -PBMA star | 5k/5k/5k | 8 | 3/3/3 | 83.9 | 49,600 | 1.17 | 9,500 |
| S4-5 | Dansyl-POEGMA-Dansyl-PQDMAEMA-Dansyl-PBMA star | 15k/15k/15k | 8 | 3/3/3 | 77.1 | 61,000 | 1.32 | 19,000 |
| S4-6 | SN38-POEGMA-SN38 - PQDMAEMA-SN38 -PBMA star | 15k/15k/15k | 8 | 3/3/3 | - | - | | - |
| S4-7 | Dansyl-POEGMA-Dansyl-PQDMAEMA-SN38-PBMA star | 15k/15k/15k | 8 | 3/3/3 | 77.4 | 67,200 | 1.60 | 21,500 |
| S4-8 | Dansyl-POEGMA-Dansyl-PQDMAEMA star | 15k/15k | 12 | 3/3 | 77.3 | 66,800 | 1.36 | 25,600 |

| | | | | | | | | |
|---|---|---|---|---|---|---|---|---|
| ^{a)} Arm conversions were calculated from GPC traces as: arm conversion = Areaₛₜₐᵣ / ( Areaₛₜₐᵣ + Area_{macro-RAFT}); ^{b)} *M*ₙ (GPC) and *Ð* (GPC) were obtained from DMAc GPC using PMMA standards. | | | | | | | | |

### Example 2

### Reductive cleavage of mikto-arm star polymers using tributylphosphine

The mikto-arm polymers containing disulfide bonds in their core (5 mg) were dissolved in 1 mL of DMAc containing 20 mg tributylphosphine. The solution was stirred at room temperature under nitrogen atmosphere for 30 minutes prior to GPC analyses. Figure 3 (IV) shows the GPC traces of the star polymer and the degraded polymer demonstrating the cleavage of the cross linked core to produce low molecular weight polymer with comparable molecular weight to that of the arms.

### Example 3

### Synthesis of mikto -arm star polymer similar to S4-1 (see Table 2) incorporating fluorescent dye rhodamine methacrylate

Mikto-arm star polymers with PolyFlour were synthesized using arm-first approach.

For the first step, a typical procedure for the synthesis of linear polymer with PolyFlour is as follows. Stock solution I of VAZO-88 (25 mg) in DMF (2.5 g) was prepared in a flask. A mixture of DTTCP (32 mg), PolyFlour (rhodamine methacrylate MA) (11 mg), OEGMA8-9 (1.5 g), stock solution I (190 mg) and DMF (1.3 g) was prepared in a second flask. This stock solution was transferred to an ampoule which was degassed by three repeated freeze-evacuate-thaw cycles and sealed under vacuum. The ampoules were heated at 90°C for the specified time and then subjected to GPC and ¹H NMR analysis.

**Table 3. The reaction time, monomer conversion and molecular weight data for the synthesis of homopolymer macro RAFT agents with PolyFluor from monomers OEGMA₈₋₉, DMAEMA and n-BMA.**

| **Sample code** | **Polymer composition** | **Time** | **Conversion (% )** | ***Mₙ* (by GPC)** | **Ð (by GPC)** |
|---|---|---|---|---|---|
| M-RAFT 4 | P(OEGMA₈₋₉-RhMA) | 4 | 75.2 | 11,100 | 1.18 |
| M-RAFT 5 | P(DMAEMA-RhMA) | 6.5 | 82.5 | 14,500 | 1.11 |
| M-RAFT 6 | P(*n*-BMA-RhMA) | 8 | 80.2 | 11,600 | 1.09 |

Then the second step, stock solutions of ACCN (1.0 wt.%), P(OEGMA₈₋₉-RhMA) (30.0 wt.%), P(DMAEMA-RhMA) (30.0 wt.%), P(*n*-BMA-RhMA) (30.0 wt.%), DSDMA (20.6 wt.%) in DMF were prepared in flasks. Specific amounts of each monomer ([Monomer]: [Polymer] =6:1) was added into polymer solution respectively. A mixture of ACCN solution (86 mg), P(OEGMA₈₋₉-RhMA) solution (111 mg), P(DMAEMA-RhMA) solution (109 mg), P(*n*-BMA-RhMA) solution (107 mg), DSDMA solution (178 mg) and DMF (284 mg) was prepared in a flask. The stock solution was transferred to an ampoule which was degassed by three freeze-evacuate-thaw cycles and sealed under vacuum. The ampoule was heated at 90 °C for 20h.

**Table 4. Monomer conversion, polymer molecular weight and dispersity of mikto-arm polymers prepared from homopolymers listed in Table 3 for cross linker to homopolymer RAFT agent ratios of 12 and 8. The molecular weight of the degradation products of the mikto-arm polymers are also shown**

| **Sample code** | **DSDMA : M-RAFT** | **Arm Conversion (% )** | ***Mₙ*** | **Ð** |
|---|---|---|---|---|
| S4-10 | 12 | 87.5 | 117,100 | 1.27 |
| Cleaved S4-10 | | | 15,000 | 1.32 |
| S4-11 | 8 | 86.9 | 78,300 | 1.20 |
| Cleaved S4-11 | | | 14,300 | 1.26 |

### Example 4

### Materials and Methods

### Cells

Chinese Hamster Ovary cells constitutively expressing Green Fluorescent protein (CHO-GFP) (CSIRO, Australia) were grown in MEMα modification supplemented with 10% foetal bovine serum, 10 mM Hepes, 0.01% penicillin and 0.01% streptomycin at 37 °C with 5% CO₂ and subcultured twice weekly.

Adenocarcinomic human alveolar basal epithelial cells (A549 ATCC No. CCL185), Human hepatoma cells (HuH7 Kindly received from VIDRL, Australia) were grown in DMEM supplemented with 10% foetal bovine serum, 10 mM Hepes, 2 mM glutamine, 0.01% penicillin and 0.01% streptomycin at 37 °C with 5% CO₂ and subcultured twice weekly.

### siRNA

The anti-GFP and negative control siRNAs were obtained from QIAGEN (USA). The anti-GFP siRNA sequence is sense 5' gcaagcugacccugaaguucau 3' and antisense 5'gaacuucagggucagcuugccg 3'. The equivalent sequence as DNA oligonuclotides are used as non-silencing controls
siFAm is the same sequence at the anti-GFP siRNA with a 5' FAM label on the sense strand.

The anti coatomer protein complex., subunit alpha (COPA) siRNA pool was purchased from Sigma Aldrich (USA). The four siRNA sequences are 1; 5'-ACUCAGAUCUGGUGUAAUA[dT][dT]-3' 2; 5'-GCAAUAUGCUACACUAUGU[dT][dT]-3' 3; 5'-GAUCAGACCAUCCGAGUGU[dT][dT]-3' 4; 5'-GAGUUGAUCCUCAGCAAUU[dT][dT]-3'.

### Binding

### Formation of polymer/siRNA complexes:

Nitrogen:Phosphate (N:P) ratios of polymer to 50 pmole siRNA or siDNA were calculated. Complexes were formed by the addition of OPTIMEM media (Invitrogen, USA) to eppendorf tubes. The required amount of polymer resuspended in water was added to the tubes and the mixture vortexed. 50 pmole of si22 or di22 was then added to the tubes and the sample vortexed. Complexation was allowed to continue for 1 h at room temperature.

### Agarose gel

Samples containing 50 pmole of siRNA were electrophoresed on a 2% agarose gel in TBE at 100V for 40 min. siRNA was visualised by gel red (Jomar Bioscience) on a UV transilluminator with camera, the image was recorded by the GeneSnap program (Syngene, USA).

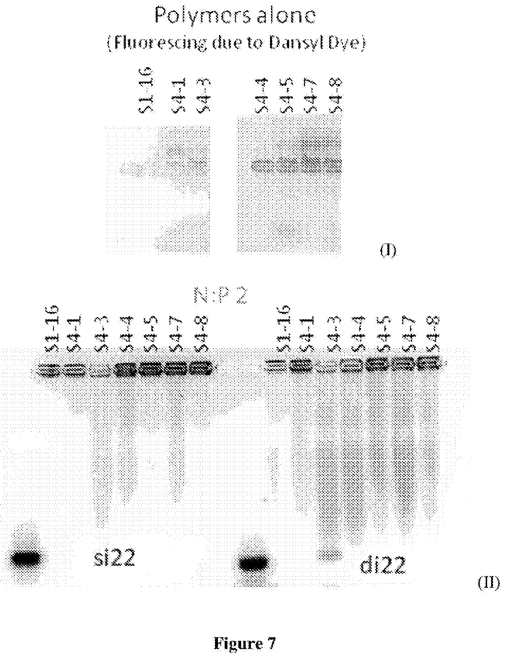

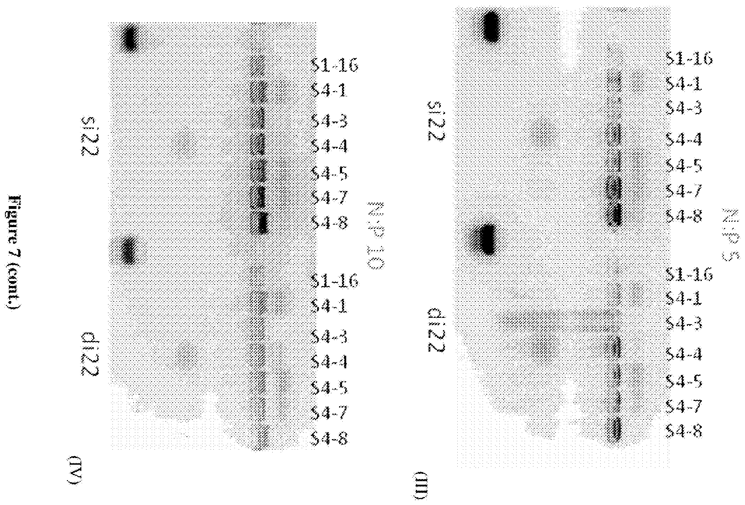

Mikto-arm polymers prepared according to the procedure in Example 1 were used in evaluation of siRNA binding, toxicity and silencing. Results of siRNA binding with mikto-arm polymers prepared in Example 1 are illustrated in Figure 7. Figure 7(I) illustrates the polymer alone whereas Figure 7(II), (III) and (VI) respectively illustrate binding for polymer:siRNA (N:P) ratios of 2, 5 and 10. The results in Figure 7 illustrates, even at low N:P ratio good siRNA binding is observed, which indicates that low amount of polymer can be used to formulate polymer/siRNA complexes to achieve good silencing.

### siRNA release by disulphide bond cleavage

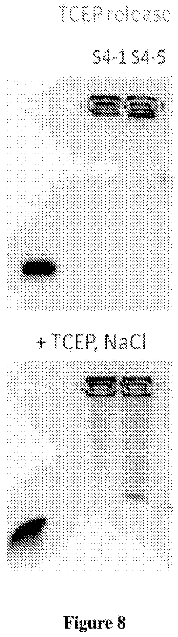

TCEP solution (50 mM) was prepared using deoxygenated water and stored at -20°C. Polymer_{/}si22 complexes (50 pmol) were assembled as described above. These polyplexes were subjected to 50 mM TCEP reduction in the presence of 0.3 M NaCl in pH5 sodium acetate buffer. Reactions were incubated at 37°C for 2 h and analysed for si22 release by electrophoresis on a 2% agarose gel as described above. Figure 8 illustrates the cleavage of the disulphide bonds in mikto-arm star polymers S4-1 and S4-5.

### Example 5

### Toxicity polymers alone

### Toxicity Assay

CHO-GFP and A549 cells were seeded at 1x10⁴ while Huh7 cells were seeded at 2x10⁴cells in 96-well tissue culture plates in triplicate and grown overnight at 37 °C with 5% CO₂.

The serially diluted polymer materials were added to 3 wells in the 96 well culture plates for each sample and incubated for 72 h at 37 °C in 200 µl standard media. Toxicity was measured using the Alamar Blue reagent (Invitrogen USA) according to manufacturer's instructions. Briefly, media was removed, cells were washed with PBS and replaced with 100 µl of standard media containing 10% Alamar Blue reagent, cells were then incubated for 4 h at 37 °C with 5% CO₂. The assay was read on an EL808 Absorbance microplate reader (BIOTEK, USA) at 540 nm and 620 nm. Cell viability was determined by subtracting the 620 nm measurement from the 540 nm measurement. Obtained data was analysed in Microsoft Excel. Results are presented as a percentage of untreated cells and the presented data are representative of three separate experiments in triplicate.

CHO-GFP and A549 cells were seeded at 1x10⁴ and Huh7 cells were seeded at 2x10⁴ cells/well in 96-well tissue culture plates in triplicate and grown overnight at 37 °C with 5% CO₂. For positive and negative controls siRNAs were transfected into cells using Lipofectamine 2000 (Invitrogen, USA) as per manufacturer's instructions. Briefly, 10 picomole of the relevant siRNA were mixed with 0.1 µl of Lipofectamine 2000 both diluted in 50 µl OPTI-MEM (Invitrogen, USA) and incubated at room temperature for 20 mins. The siNA: lipofectamine mix was added to cells and incubated for 4 h. Cell media was replaced and incubated for 72 h.

For polymer/siRNA complexes cell media was removed and replaced with 200 µl OPTI-MEM. Polymer/siRNA complexes were made as described above were added to cells and incubated for 4 h, media was replaced to standard growth media and incubated for a further 72h. Toxicity was determined using the Alamar Blue assay described above. Obtained data was analysed in Microsoft Excel. Results are presented as a percentage of untreated cells and the presented data are representative of three separate experiments in triplicate.

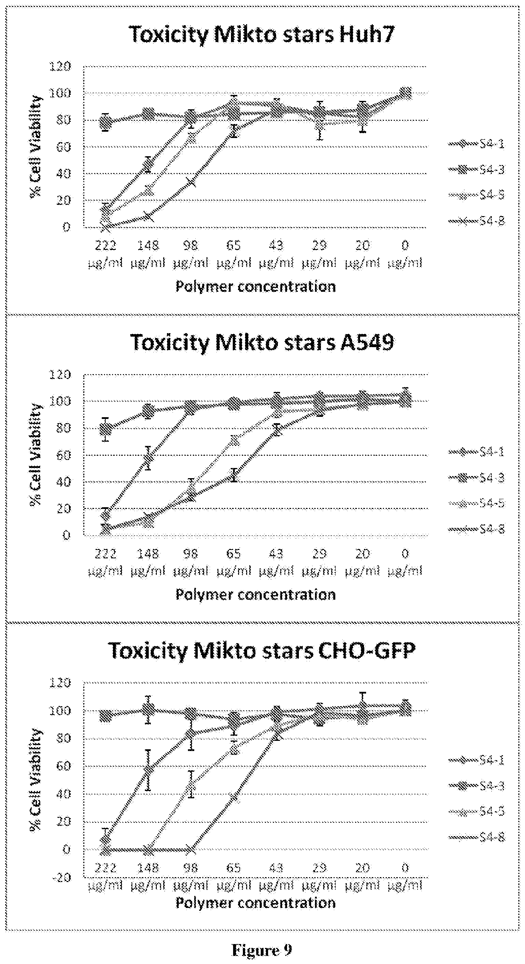

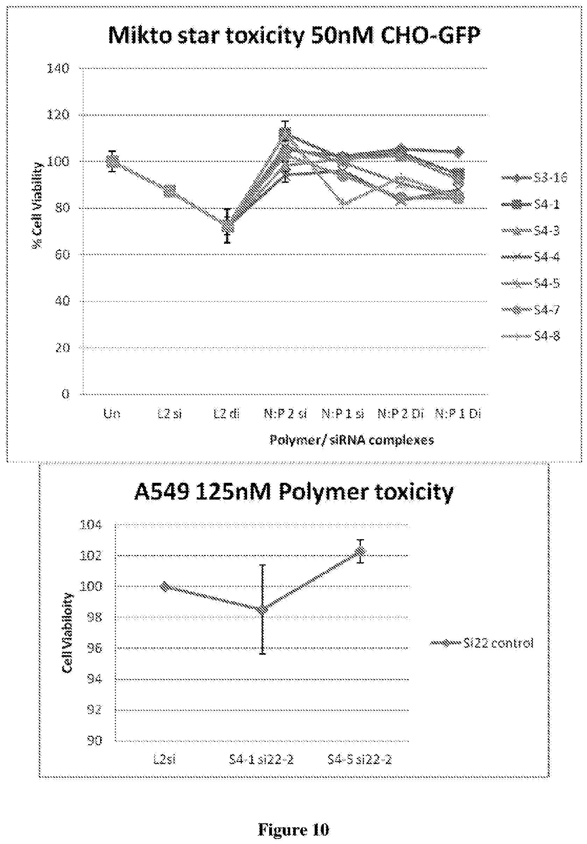

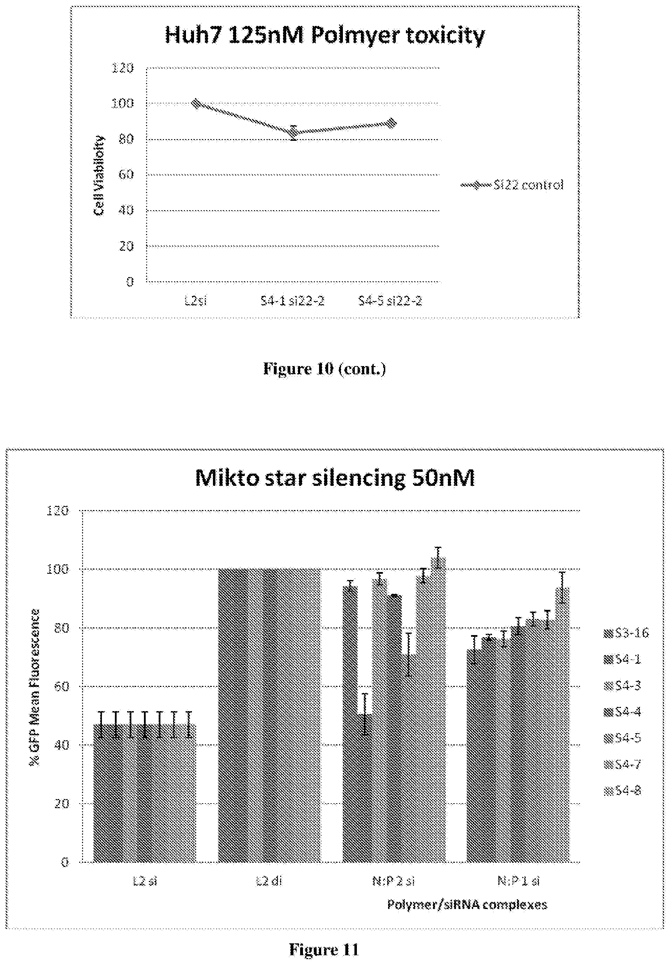

The cell viability as a function of the polymer concentration (polymer alone) is illustrated for cell lines Huh7, A549 and CHO-GFP in Figure 9, while Figure 10 shows the cell viability of the polymer/siRNA complexes in the same three cell lines.

### Example 6

### siRNA silencing CHO-GFP

CHO-GFP cells were seeded at 1x10⁴ cells in 96-well tissue culture plates in triplicate and grown overnight at 37 °C with 5% CO₂. For positive and negative controls siRNAs were transfected into cells using Lipofectamine 2000 (L2) (Invitrogen, USA) as per manufacturer's instructions. Briefly, 10 picomole of the relevant siRNA were mixed with 0.1 µl of Lipofectamine 2000 both diluted in 50 µl OPTI-MEM (Invitrogen, USA) and incubated at room temperature for 20 mins. The siNA: lipofectamine mix was added to cells and incubated for 4 h. Cell media was replaced and incubated for 72 h.

For polymer/siRNA complexes cell media was removed and replaced with 200 µl OPTI-MEM. Polymer/siRNA complexes were made as described above were added to cells and incubated for 4 h, media was replaced to standard growth media and incubated for a further 72h.

After 72hrs of incubation 96 well plate containing CHO-GFP cells were washed with PBSA and GFP fluorescence was analysed in a Synergy. HT (USA) at 516nm. Obtained data was analysed in Microsoft Excel. Results are presented as a percentage of polymer/ non-specific silencing DNA complexes and the presented data are representative of three separate experiments in triplicate.

Figure 11 illustrates the silencing of the gene responsible for producing the green fluorescent protein (GFP) by mikto-arm polymers prepared in Example 1 (Table 2).

### Silencing other cell lines

A549 cells were seeded at 1x10⁴ and Huh7 cells were seeded at 2x10⁴ cells/well in 96-well tissue culture plates in triplicate and grown overnight at 37 °C with 5% CO₂. For positive and negative controls siRNAs were transfected into cells using Lipofectamine 2000 (L2) (Invitrogen, USA) as per manufacturer's instructions. Briefly, 10 picomole of the relevant siRNA were mixed with 0.1 µl of Lipofectamine 2000 both diluted in 50 µl OPTI-MEM (Invitrogen, USA) and incubated at room temperature for 20 mins. The siNA: lipofectamine mix was added to cells and incubated for 4 h. Cell media was replaced and incubated for 72 h.

For polymer/siRNA complexes cell media was removed and replaced with 200 µl OPTI-MEM. Polymer/siRNAcomplexes were made as described above and were added to cells and incubated for 4 h, media was replaced to standard growth media and incubated for a further 72h. As COPA is an essential gene, silencing is measured by toxicity using the Alamar Blue assay described above. Obtained data was analysed in Microsoft Excel. Results are presented as a percentage of untreated cells and the presented data are representative of three separate experiments in triplicate.

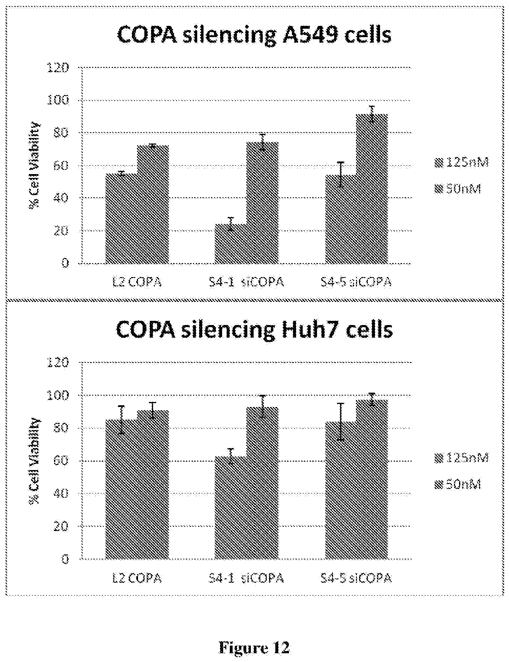

Figure 12 illustrates the COPA silencing in Huh7 and A549 cells.

### Example 7

### Delivery of cancer drug SN38

Polymers with SN38 attached covalently via a RAFT agent were tested for the ability to deliver SN38. A549 cells were seeded at 1x10 ⁴ and Huh7 cells were seeded at 2x10⁴ cells in 96-well tissue culture plates in triplicate and grown overnight at 37 °C with 5% CO₂.

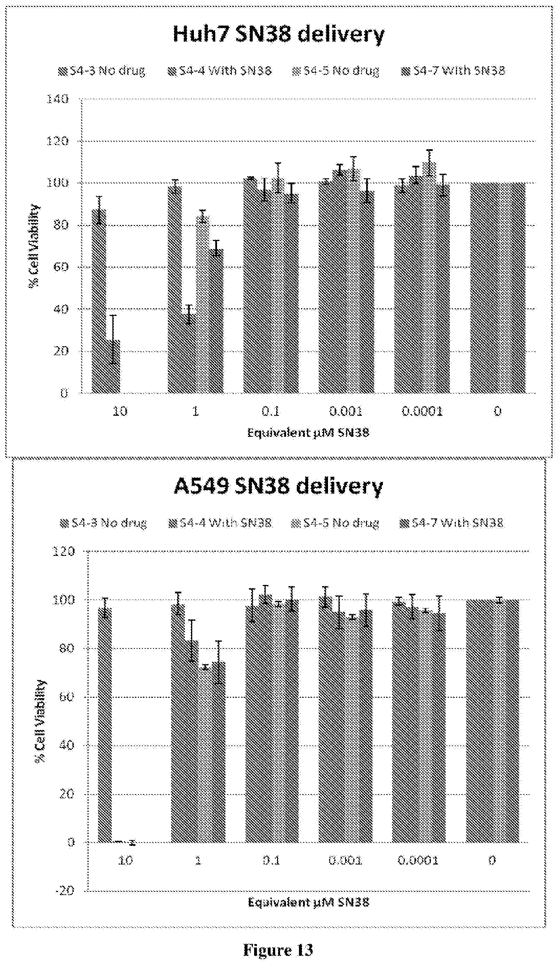

Polymer concentrations were calculated to deliver either 10, 1, 0.1 or 0.01 µM SN38. These were added to 3 wells in the 96 well culture plates for each sample and incubated for 72 h at 37 °C in 200 µl standard media. Toxicity was measured using the Alamar Blue reagent (Invitrogen USA) according to manufacturer's instructions. Briefly, media was removed, cells were washed with PBS and replaced with 100 µl of standard media containing 10% Alamar Blue reagent, cells were then incubated for 4 h at 37 °C with 5% CO₂. The assay was read on an EL808 Absorbance microplate reader (BIOTEK, USA) at 540 nm and 620 nm. Cell viability was determined by subtracting the 620 nm measurement from the 540 nm measurement. Obtained data was analysed in Microsoft Excel. Results are presented as a percentage of untreated cells and the presented data are representative of three separate experiments in triplicate. Results are summarized in Figure 13.

### Example 8

Polymer/si22-FAM complexes (50 pmol) were assembled as described above using the [6FAM] labelled si22 and used for examination under confocal microscope.

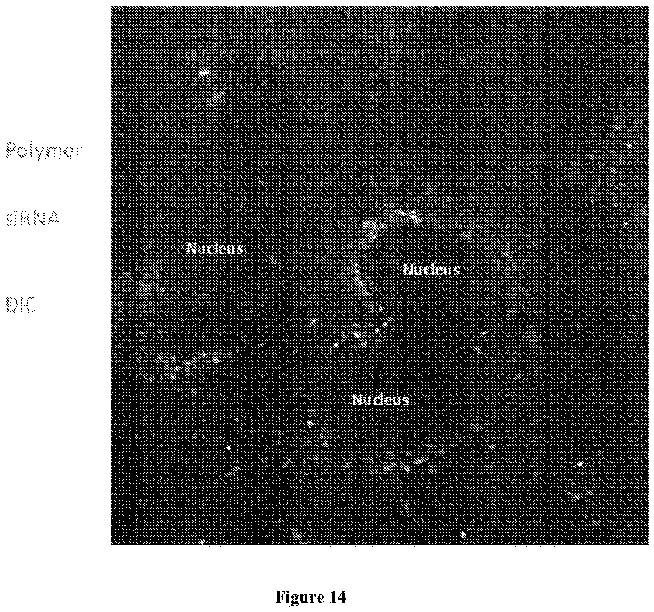

**Confocal microscopy;** Huh7 cells were seeded at 1x10⁵ cells on 13 mm round glass coverslips (Menzel, Germany) in 24 well plates (Nunc, USA).and grown overnight at 37°C with 5% CO2. For positive controls [6FAM] labelled si22 was transfected into cells using Lipofectamine 2000 (Invitrogen, USA) as per manufacturer's instructions as described below. Polymer and labelled siRNA complexes were produced as described above and added to the cells for 5 h. To process cells for confocal microscopy, cells were washed in PBS and fixed in 4% paraformadehyde (Sigma, USA) in PBS for one hour. Coverslips with cells were mounted onto slides in Vectashield (Vector Laboratories, USA). Images were acquired on a Leica SP5 confocal microscope (Leica Microsystems, Germany). Figure 14 illustrates the uptake of labelled polymer and FAM-labelled siRNA as examined by confocal microscopy.

### Example 9

Preparation of 4-arm star with block copolymer arms (comparative Example according to WO 2013/113071):

### Methods.

N,N-Dimethylaminoethyl methacrylate (DMAEMA) and oligo(ethylene glycol) methyl ether methacrylate (OEGMA₄₇₅, monomers were *polymerised via RFAT polymerisation according to the method outlined in* WO 2013/113071) to achieve a range of 4 arm stars made of block copolymers

**Table 3: Molecular weight, dispersity and composition of the star block copolymers prepared using RAFT polymerization**

| Polymer code | Mₙ (kDa) | Dispersity | Mₙ (NMR) (kDa) | Composition A:B or C*/arm | Block |
|---|---|---|---|---|---|
| TL38 PF | 30.4 | 1.22 | 45.2 | 24:16 | ABA |
| TL85 PF | 26.6 | 1.35 | 39.2 | 24:16 | ACA |

| | | | | | |
|---|---|---|---|---|---|
| *A: DMAEMA; B: OEGMA475; C: OEGMA475-BMA-DMAEMA (73.2:20:6.8) | | | | | |

### Silencing of GFP in CHO cells was carried out by a methodology identical to Example 4.

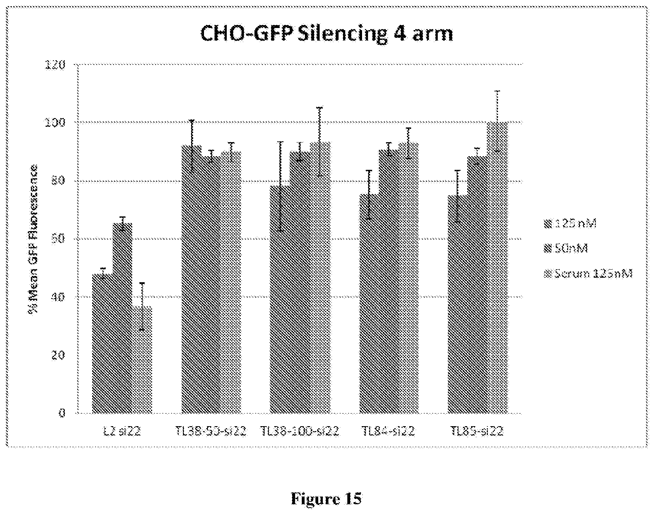

The silencing data are presented in Figure 15, illustrating CHO-GFP silencing for samples TL38-50 (50% quatemized), TL38-100 (100% quaternized). TL-84 (100% quaternized without PolyFluor), and TL-85 (100% quaternized, with Polyfluore). TL-38 polymer represents a 4-arm star prepared using a similar method to that described in WO2013/113071. The TL-84 was prepared similarly, except the arms in the 4-arm star contained cationic, hydrophilic and hydrophobic segments. The results in Figure 15 demonstrate when the arms of a star are equivalent in structure, despite having segments to represent the three homopolymers arms according to the present invention, the silencing results are inferior to those observed for mikto-arm polymers (see S4-1 in Figure 11 for comparison).

### Example 10

Mikto-arm polymers S4-1 and S4-10 used in this animal study were those disclosed in Examples 1 and Example 3, respectively.

The zeta potential of S4-1 with siRNA at N/P ratios of 2, 4 and 6 was measured at 37°C on a Malvern Zetasizer Nano Series DLS detector with a 22 mW He- Ne laser operating at 632.8 nm, an avalanche photodiode detector with high quantum efficiency, and an ALV/LSE-5003 multiple digital correlate electronics system. 24.2 uL of Mikto-arm polymer S4-1 (4 ug/uL) was added to 42 uL of water and 5 uL GFP siRNA (10 ug/uL). This mixture was vortexed and resulted in the spontaneous formation of nanoparticles. The solution was allowed to equilibrate for 2 hours at room temperature and was diluted to 1mL with 10mM NaCl immediately prior to measurement. The zeta potential of the nanoparticles with N/P of 2, 4, and 6 respectively was ∼11.6, 29.7, and 30.8.

C57/BLK6 mice were obtained from the small animal facility of Australian Animal Health Laboratory (AAHL) according to Animal Ethics committee (AEC) approval.

Mice were weighed for two days prior to intravenous injection by the tail vein with either 100 µl PBS, or 100 µl treatment in PBS.

The treatment groups consisted of:
Group 1 Control: 6 PBS control animals.
Group 2 Polymer 1 - 3: 6 animals treated with 3µg/g mouse weight Fluorescent Mikto Star (S4-10)/ siRNA targeting the ssB mRNA in PBS
Group 3 Polymer 2 - 3: 6 animals treated with 3µg/g mouse weight Mikto Star (s4-1)/siRNA targeting the ssB mRNA in PBS
Group 4 Polymer 3 - 9: 6 animals treated with 9µg/g mouse weight Fluorescent Mikto Star (S4-10)/siRNA targeting the ssB mRNA in PBS
Group 5 Polymer 1 - 9: 6 animals treated with 9µg/g mouse weight Mikto Star (s4-1)/siRNA targeting the ssB mRNA in PBS

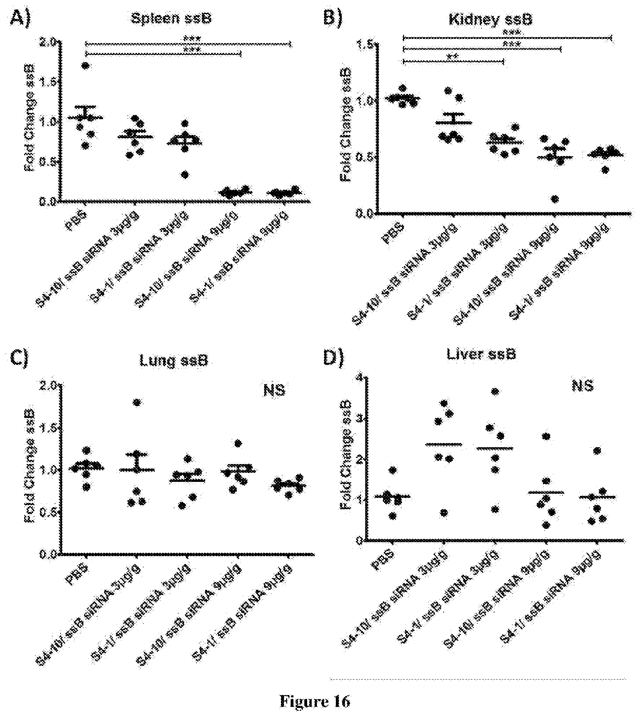

After 48 h mice were euthanasia, lung, spleen, liver and kidney tissue was collected for RNA extraction. RNA was extracted from 10mg of tissue using the Promega SimplyRNA tissue kit according to manufacturer's instructions. Quantitative real time PCR was performed for the target gene Sjögren syndrome type B antigen (ssB) (TaqMan® Gene Expression Assay, SM Catalog #: 4331182 Assay ID: Mmn00447374_m1 Gene Symbol: Ssb, mCG12976) and a house keeping gene GAPDH (TaqMan® Gene Expression Assay, SM Catalog #: 4331182 Assay ID: Mm99999915_g1 Gene Symbol: Gapdh) using the Applied Biosystems Taqman assay according to manufacturer's instructions. Data was analysed as fold change in ssB expression relative to the PBS control animals. The results are shown in Figure 16.

### Example 11

The suppliers of monomers, the abbreviations to describe monomers and polymers and other materials used in the synthesis of mikto-arm polymers described in this example are described in Example 1.

Synthesis of mikto-arm polymer to investigate the effect of p(BMA) content (TL8-1,2,3,4), is molecular weight (TL8-5,6), and the type of monomer used to prepare the core of the mikto-arm (TL8-7,8,9).

### Synthesis of mikto-arm polymers by the arm first approach

### 1) Synthesis and characterization of homopolymers of (OEGMAs₈₋₉). DMAEMA and n-BMA telechelic macroRAFT agents

In a typical polymerization experiment, 6g of OEGMA₈₋₉ monomer (1.264 × 10⁻² mol), 7.716 × 10⁻³ g of VAZO-88 initiator (3.159 × 10⁻⁵ mol), 0.12748 g of DTTCP agent (**5**) (3.154 × 10⁻⁴ mol) and 5.1063 g of DMF were weighed into a Schlenk flask. The solution mixture was degassed with four freeze-evacuate-thaw cycle and polymerized at 90°C for 4 hours.

The monomer to polymer conversion was 84 % as determined by ¹H-NMR (in CDCl₃). The conversion was calculated by comparing the integration of the COOCH_{2 of} the polymer (4.1ppm) formed to that of the COOCH₂ of the un-reacted monomer (4.3ppm). The molecular weight of the polymer calculated based on ¹H-NMR was 16.4 kDa corresponds to a degree of polymerization of 33.6. The number average molecular weight (Mₙ) of the polymer as determined by gel permeation chromatography (GPC) against linear polystyrene standards was 15.8 kDa (dispersity of 1.27). The results are summarized in Table 4.

The polymer obtained was concentrated, quaternized with methyl iodide and dialysed, The recovered polymer was lyophilized.

**Table 4. Molecular weight of mikto-arm homopolymers of OEGMA₈₋₉, DMAEMA and n-BMA telechelic macroRAFT agent**

| **Entry** | **Polymer composition** | **Time [h]** | **Conversion^{a)} [%]** | ***Mₙ* ^{b)} (Theo)** | ***Mₙ*^{c)} (GPC)** | ***Ð*^{c)} (GPC)** |
|---|---|---|---|---|---|---|
| M-RAFT 1 | P(OEGMA₈₋₉) | 4 | 84 | 1 6,364 | 15,817 | 1.27 |
| M-RAFT 2 | P(DMAEMA) | 6 | 81.3 | 15,811 | 15,484 | 1.25 |
| M-RAFT 3 | P(*n*-BMA) | 8 | 79 | 15,008 | 13,959 | 1.15 |

| | | | | | | |
|---|---|---|---|---|---|---|
| ^{a)} Monomer conversions were calculated from ¹H NMR; ^{b)} *M*ₙ (Theo) were calculated from monomer conversion; ^{c)} *M*ₙ (GPC) and *Ð* (GPC) were obtained from DMAc GPC using Polystyrene standards. | | | | | | |

### 2) Synthesis and characterization of mikto-arm polymers

A typical procedure for the synthesis of mikto-arm polymers is as follows;
Stock solutions of Vazo88 (1.0 wt.%), P(OEGMA₈₋₉) (30.0 wt.%), P(DMAEMA) (30.0 wt.%), P(*ₙ*-BMA) (30,0 wt.%), DSDMA (20.0 wt.%) in DMF were prepared. Specific amounts of each monomer ([Monomer]: [Polymer] =6:1) was added into polymer solution respectively. A mixture of Vazo88 solution (247.3 mg P(OEGMA₈₋₉) solution (539 mg), P(DMAEMA) solution (517 mg), P(*n*-BMA) solution (492 mg), DSDMA solution (528.9 mg) and DMF (18 mg) was prepared in a flask. The stock solution was transferred to an ampoule which was degassed by three freeze-evacuate-thaw cycles and sealed under vacuum. The ampoule was heated at 90 °C for 20h.

Six star polymers were prepared according to this procedure and Table 5 summarizes the relative amounts of the each homopolymer macro RAFT agents and other reagents used, and the molecular weights of the mikto-arm polymers and the cleaved mikto-arm polymers.

**Table 5. Summary of the composition, polymerization time, monomer and arm conversion and molecular weight data (DMAc using PMMA standards) for the mikto-arm star polymers prepared according to the procedure described in Example 1.**

| **Code** | **Composition** | **Arm Mn** | **[DSDMA] /[M-CTA]** | **M-CTA ratio** | **Arm conver-sion** ^{a} | **Mn (star) ^{b}** | **PDI** | **Mn (star cleaved) PD** |
|---|---|---|---|---|---|---|---|---|
| TL8-1 | POEGMA-PQDMAEMA -PBMA star | 15k/15k/15k | 12 | 3/3/3 | 78.2 | 154k | 1.36 | 22.6k |
| | | | | | | | | 1.39 |
| TL8-2 | POEGMA-PQDMAEMA -PBMA star | 15k/15k/15k | 12 | 3/3/1 | 72.7 | 157k | 1.44 | 22.4k |
| | | | | | | | | 1.47 |
| TL8-3 | POEGMA-PQDMAEMA -PBMA star | 15k/15k/15k | 12 | 3/3/5 | 77.4 | 151k | 1.42 | 22.6k |
| | | | | | | | | 1.47 |
| TL8-4 | POEGMA-PQDMAEMA star | 15k/15k | 12 | 3/3 | 70.9 | 168k | 1.52 | 22.9k |
| | | | | | | | | 1.5 |
| TL8- 5 | POEGMA-PQDMAEMA -PBMA star | 15k/5k/15k | 12 | 3/3/9 | 81.8 | 218k | 1.13 | 19.2k |
| | | | | | | | | 1.53 |
| TL8-6 | POEGMA-PQDMAEMA -PBMA star | 15k/15k/30k | 12 | 3/3/1,3 | 54.4 | 260k | 1.24 | 21.6k |
| | | | | | | | | 1.66 |
| TL8-7 | POEGMA-PQDMAEMA -PBMA star | 15k/15k/15k | 12 | 3/3/3 | 68 | 68k | 1.47 | 24.9k |
| | | | | **c** | | | | 1.63 |
| TL8-8 | POEGMA-PQDMAEMA -PBMA star | 15k/15k/15k | 12 | 3/3/3 | 75 | 206k | 1.52 | 21.6k |
| | | | | **d** | | | | 1.48 |
| TL8-9 | POEGMA-PQDMAEMA -PBMA star | 15k/15k/15k | 12 | 3/3/3 | 77.3 | 226k | 1.65 | 23.2k |
| | | | | **e** | | | | 1.49 |

| | | | | | | | | |
|---|---|---|---|---|---|---|---|---|
| ^{a)} Arm conversions were calculated from GPC traces as: arm conversion = Areaₛₜₐᵣ / (Areaₛₜₐᵣ + Area_{macro-RAFT}); ^{b)} *M*ₙ (GPC) and *Ð* (GPC) were obtained from DMAc GPC using polystyrene standards; ^{c)} Core monomer was OEGMA only; ^{d)} Core monomer was DMAEMA only; ^{e)} Core monomer was n-BMA only | | | | | | | | |

### Quaternization of the mikto-arm star polymers:

To quaternize the tertiary amino group of P(DMAEMA) arm of the mikto-arm star polymers, a stock solution of the stars mention above was diluted with DMF, then an excess of MeI was added into the solution and stirred for 16 h at room temperature. Finally, the excess of MeI was removed on a rotary evaporator; the DMF was removed by dialysis of the star polymers against water for 4 days (molecular weight membrane cut off 25.000 Da). The star polymers containing quaternized P(DMAEMA) were obtained after freeze-drying. Figure 6 shows the ¹H-NMR spectrum of the quaternized polymer.

### Reductive cleavage of mikto-arm star polymers using tributylphosphine

The mikto-arm polymers containing disulfide bonds in their core (5 mg) were dissolved in 1 mL of DMAc containing 20 mg tributylphosphine. The solution was stirred at room temperature under nitrogen atmosphere for 30 minutes prior to GPC analyses. Figure 3 (IV) shows the GPC traces of the star polymer and the degraded polymer demonstrating the cleavage of the cross linked core to produce low molecular weight polymer with comparable molecular weight to that of the arms.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

The reference in this specification to any prior publication (or information derived from it). or to any matter which is known, is not, and should not be taken as an acknowledgment or admission or any form of suggestion that that prior publication (or information derived from it) or known matter forms part of the common general knowledge in the field of endeavour to which this specification relates.

## Claims

1. Mikto-arm branched polymer comprising a support moiety in the form of a crosslinked polymer support moiety and at least three homopolymer chains each covalently coupled to and extending from the support moiety, wherein the at least three homopolymer chains include a cationic homopolymer chain, a hydrophilic homopolymer chain, and a hydrophobic homopolymer chain.

2. The branched polymer according to claim 1, wherein one or more of the at least three homopolymer chains is covalently coupled to the support moiety through a degradable functional group.

3. The branched polymer according to any one of claims 1 to 2 in the form of a star polymer.

4. The branched polymer according to any one of claims 1 to 3, wherein the crosslinked polymer support moiety comprises a polymerised residue of one or more multifunctional monomer selected from disulfide dimethacrylate, ethylene glycol di(meth)acrylate, triethylene glycol di(meth)acrylate, tetraethylene glycol di(meth)acrylate, 1,3-butylene glycol di(meth)acrylate, trimethylolpropane tri(meth)acrylate, 1,4-butanediol di(meth)acrylate, neopentyl glycol di(meth)acrylate, 1,6-hexanediol di(meth)acrylate, 1,6-hexanediol ethoxylated diacrylate (HEDA), pentaerythritol di(meth)acrylate, pentaerythritol tri(meth)acrylate, pentaerythritol tetra(meth)acrylate, glycerol di(meth)acrylate, glycerol allyloxy di(meth)acrylate, 1,1,1-tris(hydroxymethyl)ethane di(meth)acrylate, 1,1,1-tris(hydroxymethyl)ethane tri(meth)acrylate, 1,1,1-tris(hydroxymethyl)propane di(meth)acrylate, 1,1,1-tris(hydroxymethyl)propane tri(meth)acrylate, triallyl cyanurate, triallyl isocyanurate, triallyl trimellitate, diallyl phthalate, diallyl terephthalte, divinyl benzene, methylol (meth)acrylamide, triallylamine, oleyl maleate, glyceryl propoxy triacrylate, allyl methacrylate, methacrylic anhydride, methylenebis (meth) acrylamide, but-2-ene-1,4-diacrylate, bisphenol A ethoxylated diacrylate, N,N'- bis(acryloyl) cystanimine, divinyl adipate, 4,4'-divinylbiphenyl and N,N'-methylenebisacrylamide.

5. The branched polymer according to any one of claims 1 to 4, wherein the cationic homopolymer chain comprises a polymerised residue of one or more monomers selected from N,N-dimethyaminoethyl methacrylate, N,N-diethyaminoethyl methacrylate, N,N-dimethylaminoethyl acrylate, N,N-diethylaminoethyl acrylate, 2-aminoethyl methacrylate hydrochloride, N-[3-(N,N-dimethylamino)propyl]methacrylamide, N-(3-aminopropyl)methacrylamide hydrochloride, N-[3-(N,N-dimethylamino)propyl] acrylamide, N-[2-(N,N-dimethylamino)ethyl]methacrylamide, 2-N-morpholinoethyl acrylate, 2-N-morpholinoethyl methacrylate, 2-(N,N-dimethylamino)ethyl acrylate , 2-(N,N-dimethylamino)ethyl methacrylate, 2-(N,N-diethylamino)ethyl methacrylate, 2-acryloxyyethyltrimethylammonium chloride, mthacrylamidopropyltrimethylammonium chloride, 2-(tert-butylamino)ethyl methacrylate, allyldimethylammonium chloride, 2-(dethylamino)ethylstyrene, 2-vinylpyridine, and 4-vinylpyridine; the hydrophilic homopolymer chain comprises a polymerised residue of one or more monomers selected from acrylic acid, methacrylic acid, hydroxyethyl methacrylate, hydroxypropyl methacrylate, oligo(alkylene glycol)methyl ether (meth)acrylate (OAG(M)A), acrylamide and methacrylamide, hydroxyethyl acrylate, N-methylacrylamide, N,N-dimethylacrylamide, N,N-dimethylaminoethyl methacrylate, N,N-dimethylaminopropyl methacrylamide, N-hydroxypropyl methacrylamide, 4-acryloylmorpholine, 2-acrylamido-2-methyl-1-propanesulfonic acid, phosphorylcholine methacrylate and N-vinyl pyrolidone; and the hydrophobic homopolymer chain comprises a polymerised residue of one or more monomers selected from styrene, alpha-methyl styrene, butyl acrylate, butyl methacrylate, amyl methacrylate, hexyl methacrylate, lauryl methacrylate, stearyl methacrylate, ethyl hexyl methacrylate, crotyl methacrylate, cinnamyl methacrylate, oleyl methacrylate, ricinoleyl methacrylate, cholesteryl methacrylates, cholesteryl acrylate, vinyl butyrate, vinyl tert-butyrate, vinyl stearate and vinyl laurate.

6. A method of preparing mikto-arm branched polymer comprising a support moiety and at least three homopolymer chains each covalently coupled to and extending from the moiety, wherein the at least three homopolymer chains include a cationic homopolymer chain, a hydrophilic homopolymer chain, and a hydrophobic homopolymer chain, the method comprising:
(i) providing a cationic homopolymer chain, a hydrophilic homopolymer chain, and a hydrophobic homopolymer chain, each homopolymer chain having a living polymerisation moiety covalently coupled thereto; and
(ii) polymerising one or more multi-ethylenically unsaturated monomers under the control of the living polymerisation moieties so as to form a crosslinked polymer support moiety to which is covalently attached each of the cationic, hydrophilic, and hydrophobic homopolymer chains.

7. A complex comprising a mikto-arm branched polymer and a nucleic acid molecule, the branched polymer comprising a support moiety in the form of a crosslinked polymer support moiety and at least three homopolymer chains each covalently coupled to and extending from the moiety, wherein the at least three homopolymer chains include a cationic homopolymer chain, a hydrophilic homopolymer chain, and a hydrophobic homopolymer chain.

8. The complex according to claim 7, wherein the at least three homopolymer chains include
(i) a cationic homopolymer chain comprising from about 10 to about 200 monomer residue units, and/or
(ii) a hydrophilic homopolymer chain comprising from about 10 to about 150 monomer residue units, and/or
(iii)a hydrophobic homopolymer chain comprising from about 15 to about 150 monomer residue units.

9. The complex according to any one of claims 7 or 8 having a Zeta potential ranging from about 10 mV to about 40 mV.

10. The complex according to any one of claims 7 to 9, wherein the nucleic acid molecule is selected from gDNA, cDNA, double or single stranded DNA oligonucleotides, sense RNAs, antisense RNAs, mRNAs, tRNAs, rRNAs, small/short interfering RNAs (siRNAs), double-stranded RNAs (dsRNA), short hairpin RNAs (shRNAs), piwi-interacting RNAs (PiRNA), micro RNA/small temporal RNA (miRNA/stRNA), small nucleolar RNAs (SnoRNAs), small nuclear (SnRNAs) ribozymes, aptamers, DNAzymes, ribonuclease-type complexes, hairpin double stranded RNA (hairpin dsRNA), miRNAs which mediate spatial development (sdRNAs), stress response RNA (srRNAs), cell cycle RNA (ccRNAs) and double or single stranded RNA oligonucleotides.

11. A method of delivering a nucleic acid molecule to a cell, the method comprising:
a) providing a complex comprising a mikto-arm branched polymer and a nucleic acid molecule, the branched polymer comprising a support moiety in the form of a crosslinked polymer support moiety and at least three homopolymer chains each covalently coupled to and extending from the moiety, wherein the at least three homopolymer chains include a cationic homopolymer chain, a hydrophilic homopolymer chain, and a hydrophobic homopolymer chain; and
b) delivering the complex to the cell.

12. A method of silencing gene expression, the method comprising transfecting a cell with a complex comprising a mikto-arm branched polymer and a nucleic acid molecule, the branched polymer comprising a support moiety in the form of a crosslinked polymer support moiety and at least three homopolymer chains each covalently coupled to and extending from the moiety, wherein the at least three homopolymer chains include a cationic homopolymer chain, a hydrophilic homopolymer chain, and a hydrophobic homopolymer chain.

13. The method according to any one of claims 11 or 12, wherein the branched polymer is in the form of a star polymer.

14. The method according to any one of claims 11 to 13, wherein at least one of the at least three homopolymer chains is conjugated with a targeting ligand, an imaging agent, a bioactive, or combination thereof.

15. The method according to any one of claims 11 to 14, wherein the complex is administered to a subject.

## Patentansprüche

1. Verzweigtes Miktoarm-Polymer, umfassend einen Trägerteil in der Form eines vernetzten Polymerträgerteils und mindestens drei Homopolymerketten, die jeweils kovalent an den Trägerteil gekoppelt sind und sich von diesem erstrecken, wobei die mindestens drei Homopolymerketten eine kationische Homopolymerkette, eine hydrophile Homopolymerkette und eine hydrophobe Homopolymerkette umfassen.

2. Verzweigtes Polymer nach Anspruch 1, wobei eine oder mehrere der mindestens drei Homopolymerketten durch eine abbaubare funktionelle Gruppe kovalent an den Trägerteil gekoppelt ist.

3. Verzweigtes Polymer nach einem der Ansprüche 1 bis 2 in der Form eines Sternpolymers.

4. Verzweigtes Polymer nach einem der Ansprüche 1 bis 3, wobei der vernetzte Polymerträgerteil einen polymerisierten Rest eines oder mehrerer multifunktioneller Monomere umfasst, ausgewählt aus Disulfiddimethacrylat, Ethylenglykoldi(meth)acrylat, Triethylenglykoldi(meth)acrylat, Tetraethylenglykoldi(meth)acrylat, 1,3-Butylenglykoldi(meth)acrylat, Trimethylolpropantri(meth)acrylat, 1,4-Butandioldi(meth)acrylat, Neopentylglykoldi(meth)acrylat, 1,6-Hexandioldi(meth)acrylat, 1,6-Hexandiol-ethoxyliertes Diacrylat (HEDA), Pentaerythritoldi(meth)acrylat, Pentaerythritoltri(meth)acrylat, Pentaerythritoltetra(meth)acrylat, Glyceroldi(meth)acrylat, Glycerolallyloxydi(meth)acrylat, 1,1,1-Tris(hydroxymethyl)ethandi(meth)acrylat, 1,1,1-Tris(hydroxymethyl)ethantri(meth)acrylat, 1,1,1-Tris(hydroxymethyl)propandi(meth)acrylat, 1,1,1-Tris(hydroxymethyl)propantri(meth)acrylat, Triallylcyanurat, Triallylisocyanurat, Triallyltrimellitat, Diallylphthalat, Diallylterephthalat, Divinylbenzol, Methylol(meth)acrylamid, Triallylamin, Oleylmaleat, Glycerylpropoxytriacrylat, Allylmethacrylat, Methacrylanhydrid, Methylenbis(meth)acrylamid, But-2-en-1,4-diacrylat, Bisphenol A-ethoxyliertes Diacrylat, N,N'-Bis(acryloyl)cystanimin, Divinyladipat, 4,4'-Divinylbiphenyl und N,N'-Methylenbisacrylamid.

5. Verzweigtes Polymer nach einem der Ansprüche 1 bis 4, wobei die kationische Homopolymerkette einen polymerisierten Rest eines oder mehrerer Monomere umfasst, ausgewählt aus N,N-Dimethylaminoethylmethacrylat, N,N-Diethylaminoethylmethacrylat, N,N-Dimethylaminoethylacrylat, N,N-Diethylaminoethylacrylat, 2-Aminoethylmethacrylathydrochlorid, N-[3-(N,N-Dimethylamino)propyl]methacrylamid, N-(3-Aminopropyl)methacrylamidhydrochlorid, N-[3-(N,N-Dimethylamino)propyl]acrylamid, N-[2-(N,N-Dimethylamino)ethyl]methacrylamid, 2-N-Morpholinoethylacrylat, 2-N-Morpholinoethylmethacrylat, 2-(N,N-Dimethylamino)ethylacrylat, 2-(N,N-Dimethylamino)ethylmethacrylat, 2-(N,N-Diethylamino)ethylmethacrylat, 2-Acryloxyethyltrimethylammoniumchlorid, Methacrylamidopropyltrimethylammoniumchlorid, 2-(tert-Butylamino)ethylmethacrylat, Allyldimethylammoniumchlorid, 2-(Diethylamino)ethylstyrol, 2-Vinylpyridin und 4-Vinylpyridin; wobei die hydrophile Homopolymerkette einen polymerisierten Rest eines oder mehrerer Monomere umfasst, ausgewählt aus Acrylsäure, Methacrylsäure, Hydroxyethylmethacrylat, Hydroxypropylmethacrylat, Oligo(alkylenglykol)methylether(meth)acrylat (OAG(M)A), Acrylamid und Methacrylamid, Hydroxyethylacrylat, N-Methylacrylamid, N,N-Dimethylacrylamid, N,N-Dimethylaminoethylmethacrylat, N,N-Dimethylaminopropylmethacrylamid, N-Hydroxypropylmethacrylamid, 4-Acryloylmorpholin, 2-Acrylamido-2-methyl-1-propansulfonsäure, Phosphorylcholinmethacrylat und N-Vinylpyrolidon; und wobei die hydrophobe Homopolymerkette einen polymerisierten Rest eines oder mehrerer Monomere umfasst, ausgewählt aus Styrol, Alpha-Methylstyrol, Butylacrylat, Butylmethacrylat, Amylmethacrylat, Hexylmethacrylat, Laurylmethacrylat, Stearylmethacrylat, Ethylhexylmethacrylat, Crotylmethacrylat, Zimtmethacrylat, Oleylmethacrylat, Ricinoleylmethacrylat, Cholesterylmethacrylaten, Cholesterylacrylat, Vinylbutyrat, Vinyltert-Butyrat, Vinylstearat und Vinyllaurat.

6. Verfahren zum Herstellen eines verzweigten Miktoarm-Polymers, umfassend einen Trägerteil und mindestens drei Homopolymerketten, die jeweils kovalent an den Teil gekoppelt sind und sich von diesem erstrecken, wobei die mindestens drei Homopolymerketten eine kationische Homopolymerkette, eine hydrophile Homopolymerkette und eine hydrophobe Homopolymerkette umfassen, wobei das Verfahren umfasst:
(i) Bereitstellen einer kationischen Homopolymerkette, einer hydrophilen Homopolymerkette und einer hydrophoben Homopolymerkette, wobei jede Homopolymerkette einen lebenden Polymerisationsteil hat, der kovalent daran gekoppelt ist; und
(ii) Polymerisieren eines oder mehrerer multiethylenisch ungesättigter Monomere unter der Kontrolle der lebenden Polymerisationsteile, um einen vernetzten Polymerträgerteil zu bilden, an den jede der kationischen, hydrophilen und hydrophoben Homopolymerketten kovalent gebunden ist.

7. Komplex, umfassend ein verzweigtes Miktoarm-Polymer und ein Nukleinsäuremolekül, wobei das verzweigte Polymer einen Trägerteil in der Form eines vernetzten Polymerträgerteils und mindestens drei Homopolymerketten umfasst, die jeweils kovalent an den Teil gekoppelt sind und sich von diesem erstrecken, wobei die mindestens drei Homopolymerketten eine kationische Homopolymerkette, eine hydrophile Homopolymerkette und eine hydrophobe Homopolymerkette umfassen.

8. Komplex nach Anspruch 7, wobei die mindestens drei Homopolymerketten umfassen:
(i) eine kationische Homopolymerkette, die von etwa 10 bis etwa 200 Monomerrest-Einheiten umfasst, und/oder
(ii) eine hydrophile Homopolymerkette, die von etwa 10 bis etwa 150 Monomerrest-Einheiten umfasst, und/oder
(iii) eine hydrophobe Homopolymerkette, die von etwa 15 bis etwa 150 Monomerrest-Einheiten umfasst.

9. Komplex nach einem der Ansprüche 7 oder 8, der ein Zeta-Potential in einem Bereich von etwa 10 mV bis etwa 40 mV hat.

10. Komplex nach einem der Ansprüche 7 bis 9, wobei das Nukleinsäuremolekül ausgewählt ist aus gDNA, cDNA, doppel- oder einzelsträngigen DNA-Oligonukleotiden, Sense-RNAs, Antisense-RNAs, mRNAs, tRNAs, rRNAs, kleinen/kurzen interferierenden RNAs (siRNAs), doppelsträngigen RNAs (dsRNAs), kurzen Haarnadel-RNAs (shRNAs), Piwi-interagierenden RNAs (PiRNAs), Mikro-RNA/kleiner temporärer RNA (miRNA/stRNA), kleinen nukleolären RNAs (SnoRNAs), kleinen nukleären (SnRNAs) Ribozymen, Aptameren, DNAzymen, Komplexen des Typs Ribonuklease, Haarnadel-Doppelstrang-RNA (Haarnadel-dsRNA), miRNAs, die die räumliche Entwicklung vermitteln (sdRNAs), Stress-Response-RNAs (srRNAs), Zellzyklus-RNA (ccRNAs) und doppel- oder einzelsträngigen RNA-Oligonukleotiden.

11. Verfahren zum Liefern eines Nukleinsäuremoleküls an eine Zelle, wobei das Verfahren umfasst:
a) Bereitstellen eines Komplexes, der ein verzweigtes Miktoarm-Polymer und ein Nukleinsäuremolekül umfasst, wobei das verzweigte Polymer einen Trägerteil in der Form eines vernetzten Polymerträgerteils und mindestens drei Homopolymerketten umfasst, die jeweils kovalent an den Teil gekoppelt sind und sich von diesem erstrecken, wobei die mindestens drei Homopolymerketten eine kationische Homopolymerkette, eine hydrophile Homopolymerkette und eine hydrophobe Homopolymerkette umfassen; und
b) Liefern des Komplexes an die Zelle.

12. Verfahren zum Ausschalten einer Genexpression, wobei das Verfahren das Transfizieren einer Zelle mit einem Komplex umfasst, der ein verzweigtes Miktoarm-Polymer und ein Nukleinsäuremolekül umfasst, wobei das verzweigte Polymer einen Trägerteil in der Form eines vernetzten Polymerträgerteils und mindestens drei Homopolymerketten umfasst, die jeweils kovalent an den Teil gekoppelt sind und sich von diesem erstrecken, wobei die mindestens drei Homopolymerketten eine kationische Homopolymerkette, eine hydrophile Homopolymerkette und eine hydrophobe Homopolymerkette umfassen.

13. Verfahren nach einem der Ansprüche 11 oder 12, wobei das verzweigte Polymer in der Form eines Sternpolymers ist.

14. Verfahren nach einem der Ansprüche 11 bis 13, wobei mindestens eine der mindestens drei Homopolymerketten mit einem Zielliganden, einem Bildgebungsmittel, einem bioaktiven Mittel, oder einer Kombination davon, konjugiert ist.

15. Verfahren nach einem der Ansprüche 11 bis 14, wobei der Komplex an einen Patienten verabreicht wird.

## Revendications

1. Polymère ramifié à ramification mikto comprenant un fragment de support sous la forme d'un fragment de support polymère réticulé et au moins trois chaînes homopolymères couplées chacune par liaison covalente au et se prolongeant à partir du fragment de support, dans lequel les au moins trois chaînes homopolymères incluent une chaîne homopolymère cationique, une chaîne homopolymère hydrophile, et une chaîne homopolymère hydrophobe.

2. Polymère ramifié selon la revendication 1, dans lequel une ou plusieurs des au moins trois chaînes homopolymères sont couplées par liaison covalente au fragment de support par l'intermédiaire d'un groupe fonctionnel dégradable.

3. Polymère ramifié selon l'une quelconque des revendications 1 à 2 sous la forme d'un polymère en étoile.

4. Polymère ramifié selon l'une quelconque des revendications 1 à 3, dans lequel le fragment de support polymère réticulé comprend un résidu polymérisé d'un ou de plusieurs monomères multifonctionnels sélectionnés parmi le diméthacrylate de disulfure, le di(méth)acrylate d'éthylène glycol, le di(méth)acrylate de triéthylène glycol, le di(méth)acrylate de tétra-éthylène glycol, le di(méth)acrylate de 1,3-butylène glycol, le tri(méth)acrylate de triméthylolpropane, le di(méth)acrylate de 1,4-butanediol, le di(méth)acrylate de néopentyl glycol, le di(méth)acrylate de 1,6-hexanediol, le diacrylate de 1,6-hexanediol éthoxylé (HEDA), le di(méth)acrylate de pentaérythritol, le tri(méth)-acrylate de pentaérythritol, le tétra(méth)acrylate de pentaérythritol, le di(méth)acrylate de glycérol, le di(méth)acrylate de glycérol allyloxy, le di(méth)-acrylate de 1,1,1-tris(hydroxyméthyl)éthane, le tri-(méth)acrylate de 1,1,1-tris(hydroxyméthyl)éthane, le di(méth)acrylate de 1,1,1-tris(hydroxyméthyl)propane, le tri(méth)acrylate de 1,1,1-tris(hydroxyméthyl)-propane, le cyanurate de triallyle, l'isocyanurate de triallyle, le trimellitate de triallyle, le phtalate de diallyle, le téréphtalate de diallyle, le divinyl benzène, le méthylol (méth)acrylamide, la triallylamine, le maléate d'oléyle, le triacrylate de glycéryl propoxy, le méthacrylate d'allyle, l'anhydride méthacrylique, le méthylènebis(méth)acrylamide, le but-2-ène-1,4-diacrylate, le diacrylate de bisphénol A éthoxylé, la N,N'-bis(acryloyl)cystanimine, l'adipate de divinyle, le 4,4'-divinylbiphényle et le N,N'-méthylènebis-acrylamide.

5. Polymère ramifié selon l'une quelconque des revendications 1 à 4, dans lequel la chaîne homopolymère cationique comprend un résidu polymérisé d'un ou de plusieurs monomères sélectionnés parmi le méthacrylate de N,N-diméthylaminoéthyle, le méthacrylate de N,N-diéthylaminoéthyle, l'acrylate de N,N-diméthylaminoéthyle, l'acrylate de N,N-diéthyl-aminoéthyle, le chlorhydrate de méthacrylate de 2-aminoéthyle, le N-[3-(N,N-diméthylamino)propyl]-méthacrylamide, le chlorhydrate de N-(3-aminopropyl)-méthacrylamide, le N-[3-(N,N-diméthylamino)propyl] acrylamide, le N-[2-(N,N-diméthylamino)éthyl]-méthacrylamide, l'acrylate de 2-N-morpholinoéthyle, le méthacrylate de 2-N-morpholinoéthyle, l'acrylate de 2-(N,N-diméthylamino)éthyle, le méthacrylate de 2-(N,N-diméthylamino)éthyle, le méthacrylate de 2-(N,N-di-éthylamino)éthyle, le chlorure de 2-acryloxyéthyl-triméthylammonium, le chlorure de méthacrylamidopropyl-triméthylammonium, le méthacrylate de 2-(tert-butyl-amino)éthyle, le chlorure d'allyldiméthylammonium, le 2-(déthylamino)éthylstyrène, la 2-vinylpyridine, et la 4-vinylpyridine ; la chaîne homopolymère hydrophile comprend un résidu polymérisé d'un ou de plusieurs monomères sélectionnés parmi l'acide acrylique, l'acide méthacrylique, le méthacrylate d'hydroxyéthyle, le méthacrylate d'hydroxypropyle, un (méth)acrylate d'oligo(alkylène glycol)méthyl éther (OAG(M)A), l'acrylamide et le méthacrylamide, l'acrylate d'hydroxyéthyle, le N-méthylacrylamide, le N,N-di-méthylacrylamide, le méthacrylate de N,N-diméthylamino-éthyle, le N,N-diméthylaminopropyl méthacrylamide, le N-hydroxypropyl méthacrylamide, la 4-acryloylmorpholine, l'acide 2-acrylamido-2-méthyl-1-propanesulfonique, le méthacrylate de phosphorylcholine et la N-vinyl pyrrolidone ; et la chaîne homopolymère hydrophobe comprend un résidu polymérisé d'un ou de plusieurs monomères sélectionnés parmi le styrène, l'alpha-méthyl styrène, l'acrylate de butyle, le méthacrylate de butyle, le méthacrylate d'amyle, le méthacrylate d'hexyle, le méthacrylate de lauryle, le méthacrylate de stéaryle, le méthacrylate d'éthylhexyle, le méthacrylate de crotyle, le méthacrylate de cinnamyle, le méthacrylate d'oléyle, le méthacrylate de ricinoléyle, les méthacrylates de cholestéryle, l'acrylate de cholestéryle, le butyrate de vinyle, le tert-butyrate de vinyle, le stéarate de vinyle et le laurate de vinyle.

6. Procédé de préparation d'un polymère ramifié à ramification mikto comprenant un fragment de support et au moins trois chaînes homopolymères couplées chacune par liaison covalente au et se prolongeant à partir du fragment, dans lequel les au moins trois chaînes homopolymères incluent une chaîne homopolymère cationique, une chaîne homopolymère hydrophile, et une chaîne homopolymère hydrophobe, le procédé comprenant :
(i) la fourniture d'une chaîne homopolymère cationique, d'une chaîne homopolymère hydrophile, et d'une chaîne homopolymère hydrophobe, chaque chaîne homopolymère ayant un fragment de polymérisation vivant couplé par liaison covalente à celle-ci ; et
(ii) la polymérisation d'un ou de plusieurs monomères à multi-insaturation éthylénique sous le contrôle des fragments de polymérisation vivants afin de former un fragment de support polymère réticulé auquel est fixée par liaison covalente chacune des chaînes homopolymères cationique, hydrophile, et hydrophobe.

7. Complexe comprenant un polymère ramifié à ramification mikto et une molécule d'acide nucléique, le polymère ramifié comprenant un fragment de support sous la forme d'un fragment de support polymère réticulé et au moins trois chaînes homopolymères couplées chacune par liaison covalente au et se prolongeant à partir du fragment, dans lequel les au moins trois chaînes homopolymères incluent une chaîne homopolymère cationique, une chaîne homopolymère hydrophile, et une chaîne homopolymère hydrophobe.

8. Complexe selon la revendication 7, dans lequel les au moins trois chaînes homopolymères incluent
(i) une chaîne homopolymère cationique comprenant environ 10 à environ 200 motifs de résidu monomère, et/ou
(ii) une chaîne homopolymère hydrophile comprenant environ 10 à environ 150 motifs de résidu monomère, et/ou
(iii) une chaîne homopolymère hydrophobe comprenant environ 15 à environ 150 motifs de résidu monomère.

9. Complexe selon l'une quelconque des revendications 7 ou 8 présentant un potentiel zêta allant d'environ 10 mV à 40 mV.

10. Complexe selon l'une quelconque des revendications 7 à 9, dans lequel la molécule d'acide nucléique est sélectionnée parmi un ADNg, un ADNc, les oligonucléotides d'ADN à double ou simple brin, les ARN sens, les ARN antisens, les ARNm, les ARNt les ARNr, les ARN interférents petits/courts (ARNsi), les ARN à double brin (ARNdb), les ARN courts en épingle à cheveux (ARNsh), les ARN interagissant avec les piwi (ARNPi), un micro-ARN/petit ARN temporel (miARN/stARN), les petits ARN nucléolaires (SnoARN), les petits ribozymes nucléaires (SnARN) les aptamères, les ADNzymes, les complexes de type ribonucléase, un ARN double brin en épingle à cheveux (ARNdb en épingle à cheveux), les miARN qui médient un développement spatial (sdARN), un ARN de réponse au stress (srARN), un ARN de cycle cellulaire (ARNcc) et les oligonucléotides d'ARN à double ou simple brin.

11. Procédé d'administration d'une molécule d'acide nucléique à une cellule, le procédé comprenant :
a) la fourniture d'un complexe comprenant un polymère ramifié à ramification mikto et une molécule d'acide nucléique, le polymère ramifié comprenant un fragment de support sous la forme d'un fragment de support polymère réticulé et au moins trois chaînes homopolymères couplées chacune par liaison covalente au et se prolongeant à partir du fragment, dans lequel les au moins trois chaînes homopolymères incluent une chaîne homopolymère cationique, une chaîne homopolymère hydrophile, et une chaîne homopolymère hydrophobe ; et
b) l'administration du complexe à la cellule.

12. Procédé d'extinction de l'expression d'un gène, le procédé comprenant la transfection d'une cellule avec un complexe comprenant un polymère ramifié à ramification mikto et une molécule d'acide nucléique, le polymère ramifié comprenant un fragment de support sous la forme d'un fragment de support polymère réticulé et au moins trois chaînes homopolymères couplées chacune par liaison covalente au et se prolongeant à partir du fragment, dans lequel les au moins trois chaînes homopolymères incluent une chaîne homopolymère cationique, une chaîne homopolymère hydrophile, et une chaîne homopolymère hydrophobe.

13. Procédé selon l'une quelconque des revendications 11 ou 12, dans lequel le polymère ramifié est sous la forme d'un polymère en étoile.

14. Procédé selon l'une quelconque des revendications 11 à 13, dans lequel au moins l'une des au moins trois chaînes homopolymères est conjuguée à un ligand de vectorisation, un agent d'imagerie, un agent bioactif, ou une combinaison de ceux-ci.

15. Procédé selon l'une quelconque des revendications 11 à 14, dans lequel le complexe est administré à un sujet.
